# EUROPEAN PATENT APPLICATION

(11) **EP 4 257 609 A1**
(43) Date of publication of application: **11.10.2023**
(21) Application number: 22167464.1
(22) Date of filing: 08.04.2022
(51) Int. Cl.: C07K 16/28, C07D 417/14, A61P 35/00, A61K 31/00, A61K 39/395

(54) **COMBINATION THERAPIES BASED ON PD-1 INHIBITORS AND SIK3 INHIBITORS**

(71) Applicant: iOmx Therapeutics AG, 82152 Martinsried (DE)
(72) Inventor: LOFERER, Hannes, 82152 Martinesried (DE); BISSINGER, Stefan, 82152 Martinsried (DE)
(74) Representative: Kuttenkeuler, David

(57) **Abstract**

The invention relates to a use of combinations of PD-1 inhibitor therapy with certain kinase inhibitors, in particular inhibitors of protein kinases including the SIK-family, CSF1R, ABL/BCR-ABL, SRC, HCK, PDGFR, KIT and/or their mutants, to overcome resistance to PD-1/PDL1 inhibitor therapy in tumours. A preferred tumour disease treatable in accordance with the invention is a squamous cell lung cancer with a PD-1 inhibitor therapy resistance phenotype.

## Description

The invention relates to a use of combinations of PD-1 inhibitor therapy with certain kinase inhibitors, in particular inhibitors of protein kinases including the SIK-family, CSF1R, ABL/BCR-ABL, SRC, HCK, PDGFR, KIT and/or their mutants, to overcome resistance to PD-1/PD-L1 inhibitor therapy in tumours. A preferred tumour disease treatable in accordance with the invention is a squamous cell lung cancer with a PD-1 inhibitor therapy resistance phenotype.

PD-1 expression on T-cells is rapidly induced after antigen exposure, following engagement between the T-cell receptor (TCR) and its cognate epitope-loaded MHC molecule in the draining lymph nodes. In addition to TCR-dependence, the presence of interleukin 2 (IL-2), IL-7, IL-15, vascular endothelial growth factor (VEGF), IL-6, and transforming growth factor beta (TGF-b) in the local cytokine milieu in lymph nodes and diseased tissue additionally contribute to PD-1 up- regulation on T-cells. PD-1 interacts with its ligands, namely PD-L1(PD-L1/B7-H1/CD274) and PD-L2 (PDL2/B7-DC/CD273). The PD-1 ligands are expressed on transformed cells, professional antigen-presenting cells (pAPCs), and epithelial cells, as well as T-cells. This interaction provides signals that tolerize T-cells to their antigenic targets, disarming their effector functions. Type 1 interferons (IFN-a/b) and tumor necrosis factor alpha (TNF-a) can up-regulate PD-L1 expression on T-cells, B-cells, natural killer (NK) cells, myeloid cells, and epithelial cells, while PD-L2 expression is inducible via interferon gamma (IFN-g), granulocyte-macrophage colony- stimulating factor (GM-CSF), and IL-4 signaling. By targeting factors that foster the development and maintenance of an immunosuppressive microenvironment, e.g. within tumors, immunotherapies can release the brakes on the host's own immune system and possibly cure of disease.

In practice, checkpoint inhibitors targeting PD-1 or its ligand PD-L1 have demonstrated unprecedented clinical efficacy in more than 15 cancer types including melanoma, non-small cell lung cancer (NSCLC), renal cell carcinoma (RCC), bladder carcinoma and Hodgkin's lymphoma. Indeed, therapeutic mAbs targeting either PD-1 or PD-L1 block ligand/receptor interactions to release T-cells from their exhausted phenotype and allow for reinvigoration of tumor antigen-specific immunity (for a review, see O'Donnell e/ a/., Cancer treatment Reviews 52 (2017), 71-81).

Nevertheless, primary resistance to anti-PD-1 therapies is a common phenomenon, affecting up to 60% of patients in some cancer types. Furthermore, it is now becoming apparent that encouraging initial responses observed amongst some patients can be undone by their development of acquired resistance to anti-PDI therapies (referred to throughout as acquired resistance) leading to disease relapse. A substantial effort is currently underway to fully elucidate the mechanisms by which anti-PD-1/PD-L1 therapies exert their efficacy, to understand mechanisms of resistance present within some patients that limit their activity, and to develop a priori combination therapeutic approaches to sensitize resistant patients. In practice and as reviewed in O'Donnell et al. (Cancer treatment Reviews 52 (2017), 71- 81), different combination strategies have been proposed or developed to augment the therapeutic activity of anti-PD-1/PD-L1 antibodies, including but not limited to: anti-CTLA4 therapy, anti-OX40 (CD134) therapy, anti-TIM3, anti-LAG3 and anti-TIGIT therapies, radiotherapy, chemo-therapies such as doxorubicin, BRAF/MEK inhibitors, cytokine therapy such as anti-VEGF mAbs (bevacizumab), anti-CD40, anti-CD73 or anti-CD137 antibodies, viral therapies using oncolytic viruses, and many more.

In particular immunotherapy such as a therapy with inhibitors of the PD-1/PD-L1 axis is now the preferred treatment for most lung cancer patients. While such therapies are used to treat unresectable stage III non-small-cell lung cancer and is the first-line therapy for non-oncogene-driven advanced/metastatic non-small-cell lung cancer patients (either alone or in combination with chemotherapy), unfortunately, most patients that respond initially to immunotherapy develop resistance over time, thus limiting the durability of immunotherapy.

It is therefore an object of the invention to provide treatment options to overcome PD-1/PD-L1 resitant phenotypes in cancer therapy.

### SUMMARY OF THE INVENTION

Generally, and by way of brief description, the main aspects of the present invention can be summarised as follows:

In **a first aspect,** the present invention provides a **compound or composition for use in the treatment of a tumour** in a subject, wherein the subject or tumour is resistant to a treatment with an inhibitor of PD-1 or of a ligand thereof; or wherein the subject was treated previously with an inhibitor of PD-1 or of a ligand thereof, and wherein the tumour is recurrent;
wherein the compound or composition is any one of:
(i) a composition comprising the inhibitor of PD-1 or of a ligand thereof (PD-1 inhibitor) and the inhibitor of Salt Inducible Kinase 3 (SIK3 inhibitor); or
(ii) a SIK3 inhibitor, and wherein the treatment comprises a concomitant or sequential administration of an inhibitor of PD-1 or of a ligand thereof; or
(iii) an inhibitor of PD-1 or of a ligand thereof, and wherein the treatment comprises a c simultaneous, separate or sequential administration of a SIK3 inhibitor.

In **a second aspect,** the present application provides **a pharmaceutical composition** comprising a inhibitor of PD-1 or of a ligand thereof (PD-1 inhibitor) and an inhibitor of Salt Inducible Kinase 3 (SIK3 inhibitor), together with a pharmaceutical acceptable carrier and/or excipient.

In **a third aspect,** the present application provides **a pharmaceutical kit,** comprising an inhibitor of PD-1 or of a ligand thereof and a SIK3 inhibitor, wherein the kit is prepared to allow for a simultaneous, separate, or sequential administration of the PD-1 inhibitor and the SIK3 inhibitor in therapy to a subject.

In **a related aspect,** the present invention provides **method for treating a tumour disease** in a subject, wherein the subject or tumour is resistant to a treatment with an inhibitor of PD-1 or of a ligand thereof; or wherein the subject was treated previously with an inhibitor of PD-1 or of a ligand thereof, and wherein the tumour is recurrent;
wherein the method comprises a step (i) or (ii):
(i) administration of a therapeutically effective amount of a composition which comprises the inhibitor of PD-1 or of a ligand thereof (PD-1 inhibitor) and the inhibitor of Salt Inducible Kinase 3 (SIK3 inhibitor) to the subject; or
(ii) administration of a therapeutically effective amount of a SIK3 inhibitor to the subject, and wherein the treatment further comprises a simultaneous, separate or sequential administration of an inhibitor of PD-1 or of a ligand thereof.

Yet **further aspects** of the invention are **disclosed** herein.

### BRIEF DESCRIPTION OF THE FIGURES

The figures show:

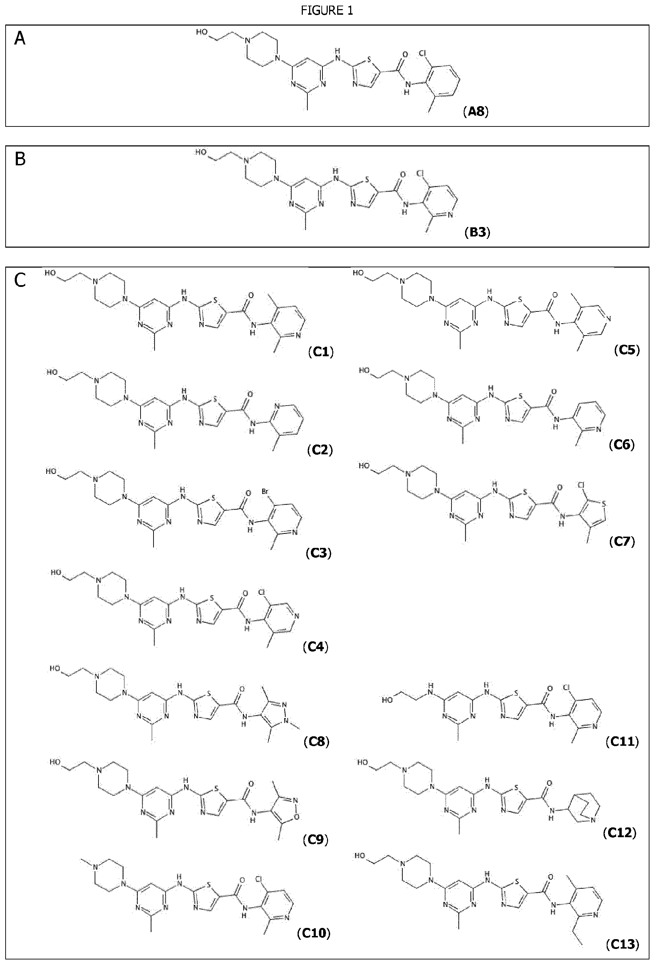

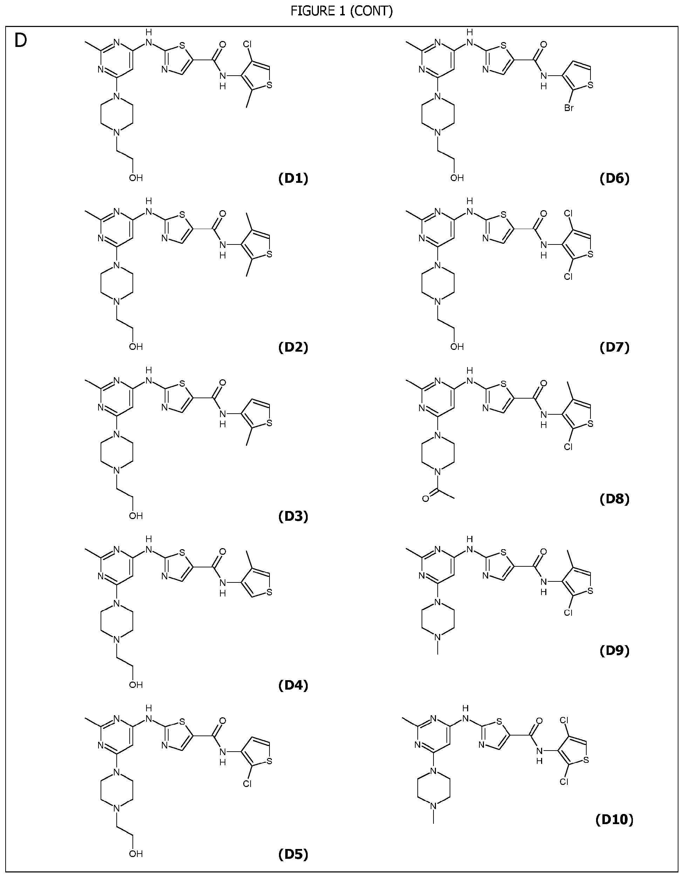

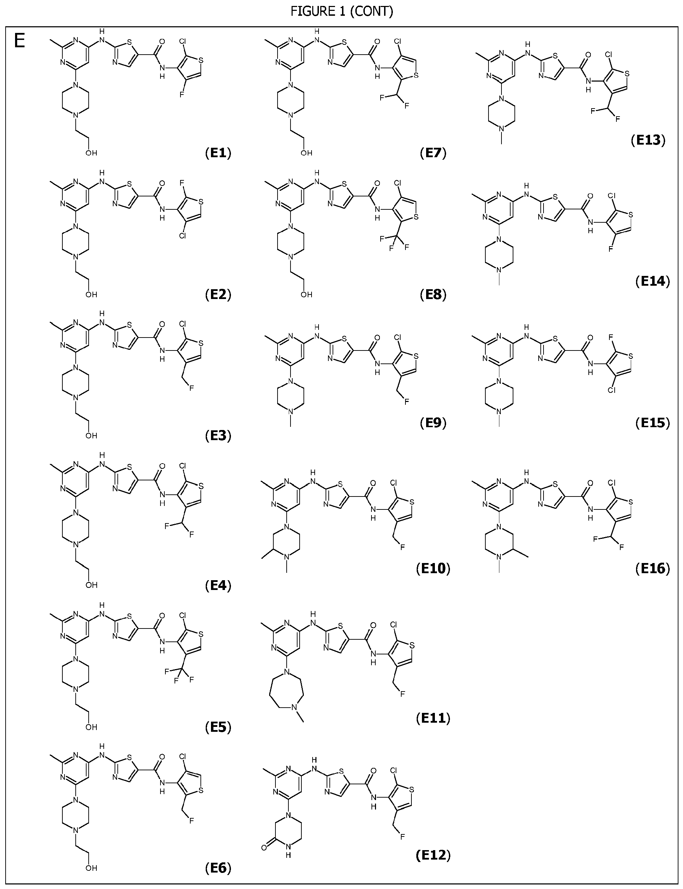

**Figure 1****:** depicts the chemical structures of: (A) dasatinib (compound A8), N-(2-chloro-6-methylphenyl)-2-((6-(4-(2-hydroxyethyl)piperazin-1-yl)-2-methylpyrimidin-4-yl)amino)thiazole-5-carboxamide; (B) the kinase inhibitor B3, N-(4-chloro-2-methylpyridin-3-yl)-2-((6-(4-(2-hydroxyethyl)piperazin-1-yl)-2-methylpyrimidin-4-yl)amino)thiazole-5-carboxamide; (C) certain other kinase inhibitors of formula (Ib)/(Ic) C1 to C13; (D) certain further kinase inhibitors of formula (Ib)/(Ic) D1 to D10; and (E) certain kinase inhibitors of formula (Ia) E1 to E16.

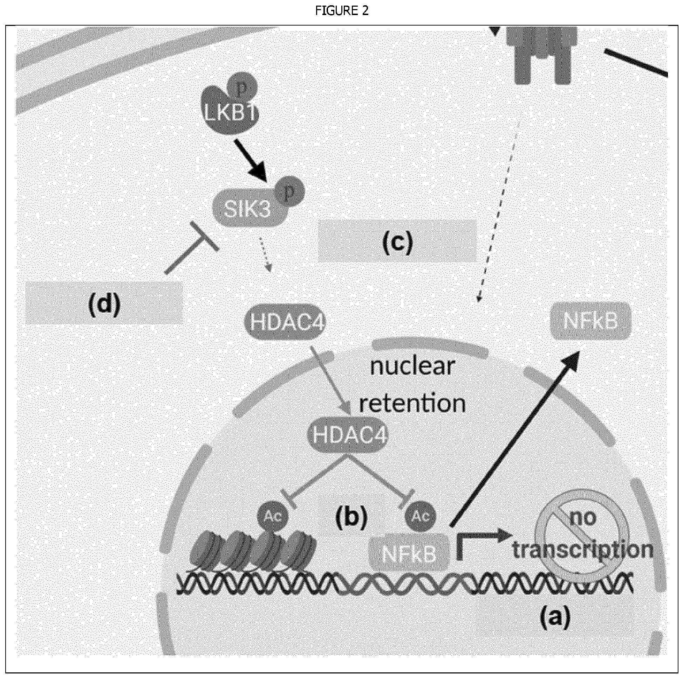

**Figure 2****:** depicts a schematic of SIK3-mediated control of the expression of survival/maintenance genes (a) by nuclear factor κB (NFκB) transcriptional regulation (b)), NFκB activity is controlled by presence in the nucleus of HDAC4 acting, as a repressive co-factor, whose retention in the cytoplasm is brought about by its phosphorylation by SIK3. Nuclear entry and presence (c) of un-phosphorylated HDAC4 (and hence reduction of expression of tumour survival/maintenance genes (a) by inhibition of the transcription-factor activity of NFκB) can be brought about by inhibition of SIK3 by a compound disclosed herein (d) such as compound E9, and thereby apoptosis is enhanced.

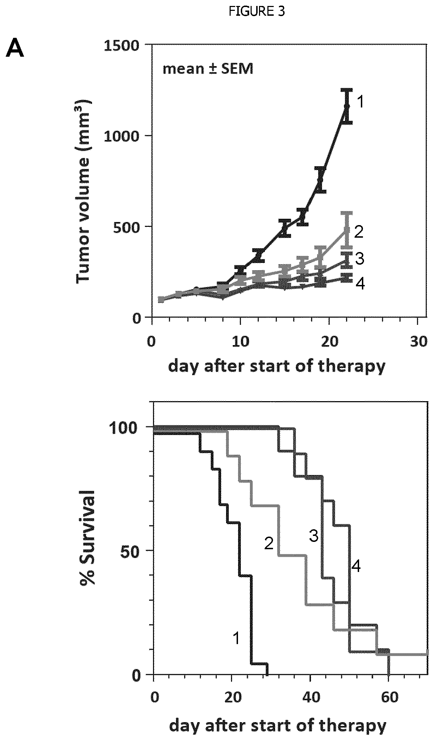

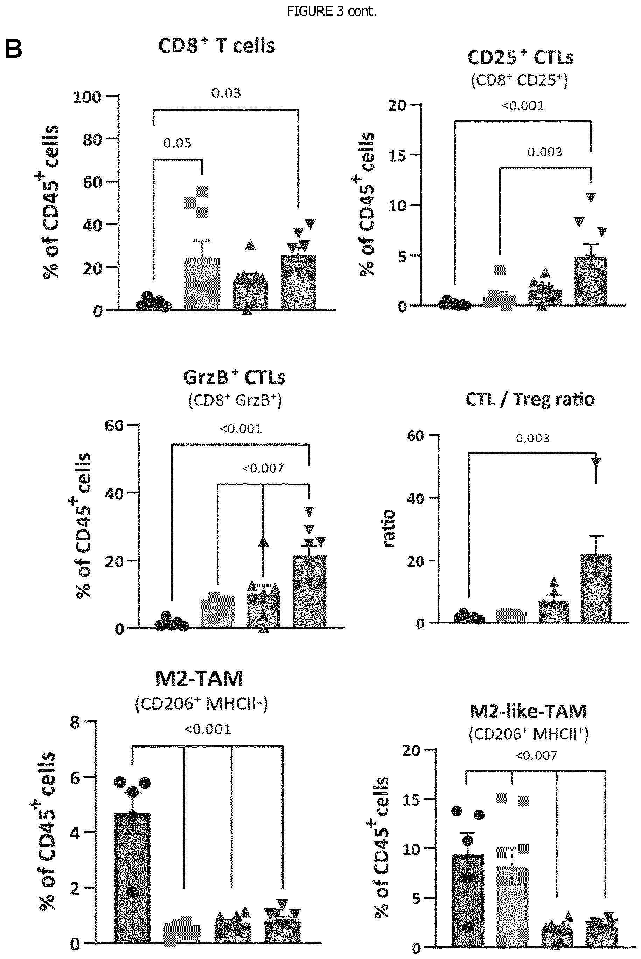

**Figure 3****:** (A) Average tumor growth kinetics and overall survival monitored for the MC38 tumor model after treatment start. Treatment regimes are indicated by indeces 1 to 4 in the figure and represent: (1) 10 ml/kg vehicle + 10 mg/kg mIgGle ctrl. (2) 10 ml/kg vehicle + 10 mg/kg a-muPD-1 (3) 25 mg/kg compound E9 + 10 mg/kg mIgGle ctrl. and (4) 25 mg/kg compound E9 + 10 mg/kg a-mu-PD-1 (B) Tumor infiltrating cytotoxic T cells (CTL), CTL to regulatory T cell ratio, tumor associated M2-macrohages (M2-TAM) and M2-like TAM were analyzed by flow cytometry.

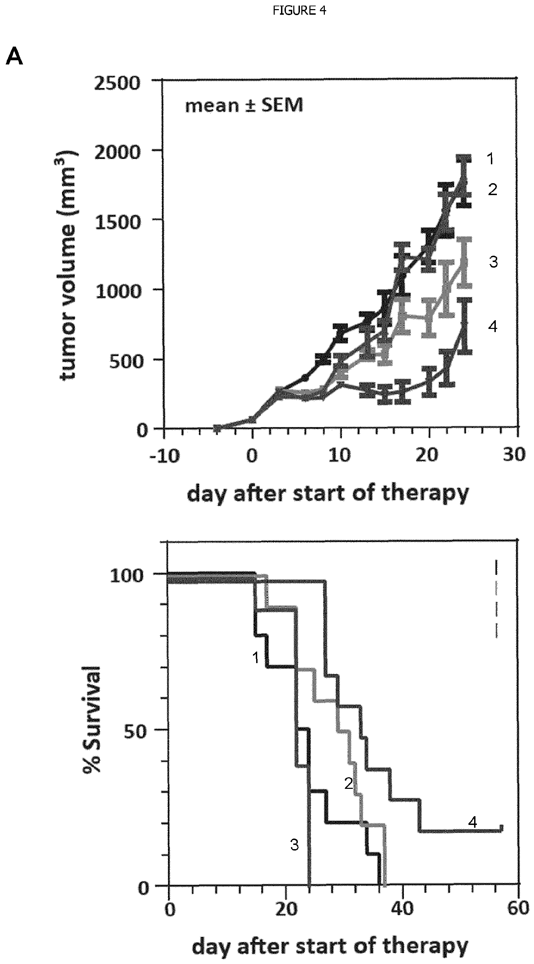

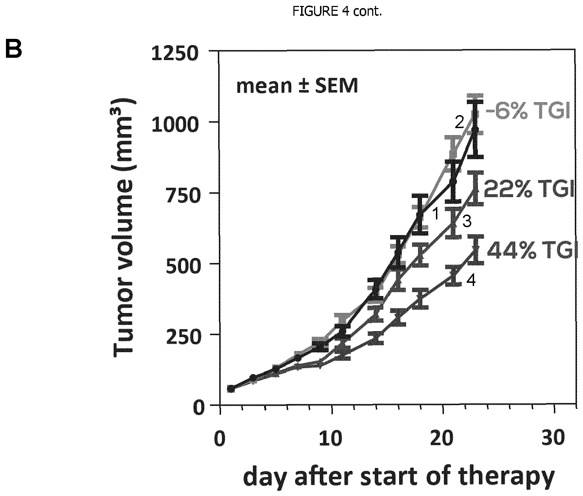

**Figure 4****:** (A) Average tumor growth kinetics and overall survival monitored for the EMT6 tumor model after treatment start. Two out often animals demonstrated complete tumor regression. Treatment regimes are indicated by indeces 1 to 4 in the figure and represent: (1) 10 ml/kg vehicle + 10 mg/kg mIgGle ctrl. (2) 10 ml/kg vehicle + 10 mg/kg a-mu-PD-1 (3) 12.5 mg/kg compound E9 + 10 mg/kg mIgGle ctrl. and (4) 12.5 mg/kg compound E9 + 10 mg/kg a-mu-PD-1 (B) Average tumor growth kinetics for the KLN205 tumor model upon initiation of therapy; Treatment regimes are indicated by indeces 1 to 4 in the figure and represent: (1) 10 ml/kg vehicle + 10 mg/kg mIgGle ctrl. (2) 10 ml/kg vehicle + 10 mg/kg a-mu-PD-1 (3) 25 mg/kg compound E9 + 10 mg/kg mIgGle ctrl. And (4) 25 mg/kg compound E9 + 10 mg/kg a-mu-PD-1.

### DETAILS OF THE PRESENT INVENTION

The present invention, and particular non-limiting aspects and/or embodiments thereof, can be described in more detail as follows.

Although the present invention may be further described in more detail, it is to be understood that this invention is not limited to the particular methodologies, protocols and reagents described herein as these may vary. It is also to be understood that the terminology used herein is for the purpose of describing particular embodiments only, and is not intended to limit the scope of the present invention which will be limited only by what is described, defined or otherwise disclosed herein, in particular in any itemised embodiments or the appended claims.

Herein, certain elements of the present invention are described in more detail. These elements are listed with specific embodiments, however, it should be understood that they may be combined in any manner and in any number to create additional embodiments. The variously described examples and preferred embodiments should not be construed to limit the present invention to only the explicitly described embodiments. This description of this application should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise. For example, if in one embodiment of the compound of the invention L is a bond and in another embodiment of the compound of the invention R³ is H, then in a preferred embodiment of the compound of the invention, L is a bond and R³ is H, or if in one embodiment of the use of a compound of the invention the subject is an adult human and in another embodiment of the use of a compound of the invention the proliferative disorder is prostate cancer, then in a preferred embodiment of the use of a compound of the invention, the subject is an adult human and the proliferative disorder is prostate cancer.

### General definitions

Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", H.G.W. Leuenberger, B. Nagel, and H. Kölbl, Eds., Helvetica Chimica Acta, CH-4010 Basel, Switzerland, (1995).

The practice of the present invention will employ, unless otherwise indicated, conventional methods of chemistry, biochemistry, and recombinant DNA techniques which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps. The term "consisting essentially of" means excluding other members, integers or steps of any essential significance or group of members, integers or steps of any essential significance. For example, a pharmaceutical composition consisting essentially of the members/components as defined herein (such as a compound as defined in any of the aspects of the invention and optionally one additional therapeutic agent) would exclude further therapeutic agents (besides the compound as defined in any of the aspects of the invention and the optional one additional therapeutic agent) but would not exclude contaminants (e.g., those from the isolation and purification method) in trace amounts (e.g., the amount of the contaminant (preferably the amount of all contaminants present in the composition) is less than 5% by weight, such as less than 4% by weight, 3% by weight, 2% by weight, 1% by weight, 0.5% by weight, 0.4% by weight, 0.3% by weight, 0.2% by weight, 0.1% by weight, 0.05% by weight, with respect to the total composition) and/or pharmaceutically acceptable excipients (such as carriers, e.g., phosphate buffered saline, preservatives, and the like). The term "consisting of" means excluding all other members, integers or steps of significance or group of members, integers or steps of significance. For example, a pharmaceutical composition consisting of the members/components as defined herein (such as a compound as defined in any of the aspects of the invention, one excipient, and optionally one additional therapeutic agent) would exclude any other compound (including a second or further excipient) in an amount of more than 2% by weight (such as any other compound in an amount of more than 1% by weight, more than 0.5% by weight, more than 0.4% by weight, more than 0.3% by weight, more than 0.2% by weight, more than 0.1% by weight, more than 0.09% by weight, more than 0.08% by weight, more than 0.07% by weight, more than 0.06% by weight, more than 0.05% by weight, more than 0.04% by weight, more than 0.03% by weight, more than 0.02% by weight, more than 0.01% by weight) with respect to the total composition. The term "comprising" encompasses the term "consisting essentially of" which, in turn, encompasses the term "consisting of'. Thus, at each occurrence in the present application, the term "comprising" may be replaced with the term "consisting essentially of" or "consisting of'. Likewise, at each occurrence in the present application, the term "consisting essentially of" may be replaced with the term "consisting of".

Where used herein, "and/or" is to be taken as specific disclosure of each of the two specified features or components with or without the other. For example, "X and/or Y" is to be taken as specific disclosure of each of (i) X, (ii) Y, and (iii) X and Y, just as if each is set out individually herein.

In the context of the present invention, the terms "about" and "approximately" are used interchangeably and denote an interval of accuracy that the person of ordinary skill will understand to still ensure the technical effect of the feature in question. The term typically indicates deviation from the indicated numerical value by ±5%, ±4%, ±3%, ±2%, ±1%, ±0.9%, ±0.8%, ±0.7%, ±0.6%, ±0.5%, ±0.4%, ±0.3%, ±0.2%, ±0.1%, ±0.05%, and for example ±0.01%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect.

The terms "a", "an" and "the" and similar references used in the context of describing the invention (especially in the context of the claims) are to be construed to cover both the singular and the plural, unless otherwise indicated herein or clearly contradicted by the context.

Recitation of ranges of values herein is merely intended to serve as a shorthand method of referring individually to each separate value falling within the range. Unless otherwise indicated herein, each individual value is incorporated into the specification as if it were individually recited herein.

All methods described herein can be performed in any suitable order unless otherwise indicated herein or otherwise clearly contradicted by the context.

The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

Several documents are cited throughout the text of this specification. Each of the documents cited herein (including all patents, patent applications, scientific publications, manufacturer's specifications, instructions, etc.), whether *supra* or *infra,* are hereby incorporated by reference in their entirety. Nothing herein is to be construed as an admission that the invention is not entitled to antedate such disclosure by virtue of prior invention.

The terms "of the [present] invention", "in accordance with the [present] invention", "according to the [present] invention" and the like, as used herein are intended to refer to all aspects and embodiments of the invention described and/or claimed herein.

It is to be understood that the application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above or below are not limited to any particular aspect or embodiment of the invention and apply equally to all aspects and embodiments that are described.

"Polymorphism" as referred to herein means that a solid material (such as a compound) is able to exist in more than one form or crystalline structure, i.e., "polymorphic modifications" or "polymorphic forms". The terms "polymorphic modifications", "polymorphic forms", and "polymorphs" are used interchangeable in the present invention. According to the present invention, these "polymorphic modifications" include crystalline forms, amorphous forms, solvates, and hydrates. Mainly, the reason for the existence of different polymorphic forms lies in the use of different conditions during the crystallization process, such as the following:
- solvent effects (the packing of crystal may be different in polar and nonpolar solvents);
- certain impurities inhibiting growth pattern and favor the growth of a metastable polymorphs;
- the level of supersaturation from which material is crystallized (in which generally the higher the concentration above the solubility, the more likelihood of metastable formation);
- temperature at which crystallization is carried out;
- geometry of covalent bonds (differences leading to conformational polymorphism);
- change in stirring conditions.

Polymorphic forms may have different chemical, physical, and/or pharmacological properties, including but not limited to, melting point, X-ray crystal and diffraction pattern, chemical reactivity, solubility, dissolution rate, vapor pressure, density, hygroscopicity, flowability, stability, compactability, and bioavailability. Polymorphic forms may spontaneously convert from a metastable form (unstable form) to the stable form at a particular temperature. According to Ostwald's rule, in general it is not the most stable but the least stable polymorph that crystallizes first. Thus, quality, efficacy, safety, processability and/or manufacture of a chemical compound, such as a compound of the present invention, can be affected by polymorphism. Often, the most stable polymorph of a compound (such as a compound of the present invention) is chosen due to the minimal potential for conversion to another polymorph. However, a polymorphic form which is not the most stable polymorphic form may be chosen due to reasons other than stability, e.g. solubility, dissolution rate, and/or bioavailability.

The term "crystalline form" of a material as used herein means that the smallest components (i.e., atoms, molecule or ions) of said material form crystal structures. A "crystal structure" as referred to herein means a unique three-dimensional arrangement of atoms or molecules in a crystalline liquid or solid and is characterized by a pattern, a set of atoms arranged in a particular manner, and a lattice exhibiting long-range order and symmetry. A lattice is an array of points repeating periodically in three dimensions and patterns are located upon the points of a lattice. The subunit of the lattice is the unit cell. The lattice parameters are the lengths of the edges of a unit cell and the angles between them. The symmetry properties of the crystal are embodied in its space group. In order to describe a crystal structure the following parameters are required: chemical formula, lattice parameters, space group, the coordinates of the atoms and occupation number of the point positions.

The term "amorphous form" of a material as used herein means that the smallest components (i.e., atoms, molecule or ions) of said material are not arranged in a lattice but are arranged randomly. Thus, unlike crystals in which a short-range order (constant distances to the next neighbor atoms) and a long-range order (periodical repetition of a basic lattice) exist, only a short-range order exists in an amorphous form.

The term "complex of a compound" as used herein refers to a compound of higher order which is generated by association of the compound with one or more other molecules. Exemplary complexes of a compound include, but are not limited to, solvates, clusters, and chelates of said compound.

The term "solvate" as used herein refers to an addition complex of a dissolved material in a solvent (such as an organic solvent (e.g., an aliphatic alcohol (such as methanol, ethanol, n-propanol, isopropanol), acetone, acetonitrile, ether, and the like), water or a mixture of two or more of these liquids), wherein the addition complex exists in the form of a crystal or mixed crystal. The amount of solvent contained in the addition complex may be stoichiometric or non-stoichiometric. A "hydrate" is a solvate wherein the solvent is water.

In isotopically labeled compounds one or more atoms are replaced by a corresponding atom having the same number of protons but differing in the number of neutrons. For example, a hydrogen atom may be replaced by a deuterium atom. Exemplary isotopes which can be used in the compounds of the present invention include deuterium, ¹¹C, ¹³C, ^{14C}, ¹⁵N, ¹⁸F, ³²P, ³²S, ³⁵S, ³⁶Cl, and ¹²⁵I.

The expression "amino protecting group" as used herein preferably refers to any group by which an amino group contained in a compound can be transferred into a less reactive (i.e., protected) amino group. Preferably, amino protecting groups can be incorporated into the corresponding compound under mild conditions, in a chemoselective and/or regioselective manner, and/or in good yields. Furthermore, the amino protecting groups should be stable under the conditions to which the protected compound is to be subjected (e.g., the conditions of the desired reaction and/or purification conditions). Preferably, the amino protecting groups should minimize the risk of racemization of a stereogenic center, when present in the compound. In one embodiment, the amino protecting groups should be removable from the protected compound under mild conditions and in a selective manner such that the deprotected compound is obtained in high yields. Exemplary amino protecting groups include tert-butyloxycarbonyl (BOC), 9-fluorenylmethoxycarbonyl (FMOC), benzyloxycarbonyl (Cbz), p-methoxybenzylcarbonyl (MOZ), acetyl (Ac), trifluoroacetyl, benzoyl (Bz), benzyl (Bn), p-methoxybenzyl (PMB), 3,4-dimethoxyphenyl (DMPM), p-methoxyphenyl (PMP), 2,2,2-trichloroethoxycarbonyl (Troc), triphenylmethyl (trityl; Tr), toluenesulfonyl (tosyl; Ts), *para-*bromophenylsulfonyl (brosyl), 4-nitrobenzenesulfonyl (nosyl), and 2-nitrophenylsulfenyl (Nps).

The term "half-life" relates to the period of time which is needed to eliminate half of the activity, amount, or number of molecules. In the context of the present invention, the half-life of a compound disclosed herein (eg a compound of formula (Ia), (Ib) or (Ic)) is indicative for the stability of said compound.

The terms "subject", "patient", "individual", or "animal" relate to multicellular animals, such as vertebrates. For example, vertebrates in the context of the present invention are mammals, birds (e.g., poultry), reptiles, amphibians, bony fishes, and cartilaginous fishes, in particular domesticated animals of any of the foregoing as well as animals (in particular vertebrates) in captivity such as animals (in particular vertebrates) of zoos. Mammals in the context of the present invention include, but are not limited to, humans, non-human primates, domesticated mammals, such as dogs, cats, sheep, cattle, goats, pigs, horses etc., laboratory mammals such as mice, rats, rabbits, guinea pigs, etc. as well as mammals in captivity such as mammals of zoos. The term "animal" as used herein also includes humans. Particular non-limiting examples of birds include domesticated poultry, and include birds such as chickens, turkeys, ducks, geese, guinea fowl, pigeons, pheasants etc.; while particular non-limiting examples of bony or cartilaginous fish include those suitable for cultivation by aquiculture, and include bony fish such as salmon, trout, perch, carp, cat-fish, etc.

In **a first aspect** the invention provides **a compound or composition for use in the treatment of a tumour** in a subject, wherein the subject or tumour is resistant to a treatment with an inhibitor of PD-1 or of a ligand thereof; or wherein the subject was treated previously with an inhibitor of PD-1 or of a ligand thereof, and wherein the tumour is recurrent;
wherein the compound or composition is any one of (i) to (iii):
(i) a composition comprising the inhibitor of PD-1 or of a ligand thereof (PD-1 inhibitor) and the inhibitor of Salt Inducible Kinase 3 (SIK3 inhibitor); or
(ii) a SIK3 inhibitor, and wherein the treatment comprises a concomitant or sequential administration of an inhibitor of PD-1 or of a ligand thereof; or
(iii) an inhibitor of PD-1 or of a ligand thereof, and wherein the treatment comprises a c simultaneous, separate or sequential administration of a SIK3 inhibitor.

In an alternative aspect thereof, the invention provides a use of the above compound or composition for the manufacture of a medicament for the treatment of a tumour in a subject, wherein the subject or tumour is resistant to a treatment with an inhibitor of PD-1 or of a ligand thereof; or wherein the subject was treated previously with an inhibitor of PD-1 or of a ligand thereof, and wherein the tumour is recurrent. In this alternative aspect the compound or composition is as defined in the first aspect.

In a further alternative aspect there is provided a method for treating a tumour disease in a subject, wherein the subject or tumour is resistant to a treatment with an inhibitor of PD-1 or of a ligand thereof; or wherein the subject was treated previously with an inhibitor of PD-1 or of a ligand thereof, and wherein the tumour is recurrent; wherein the method comprises a step (i) or (ii):
(i) administration of a therapeutically effective amount of a composition which comprises the inhibitor of PD-1 or of a ligand thereof (PD-1 inhibitor) and the inhibitor of Salt Inducible Kinase 3 (SIK3 inhibitor) to the subject; or
(ii) administration of a therapeutically effective amount of a SIK3 inhibitor to the subject, and wherein the treatment further comprises a simultaneous, separate or sequential administration of an inhibitor of PD-1 or of a ligand thereof.

The invention surprisingly identified a strong synergisit therapeutic effect in certain clinical scenarios when combining SIk3 inhibitors with PD-1 inhibitors according to the definitions herein. The invention may relate to a coadministration of SIK3 inhbitors and PD-1 inhibitors. As used herein the term "co-administering" as used herein means a process whereby the combination of the SIK3 inhibitor and the PD-1 inhibitor is administered to the same patient. The SIK3 inhibitor and the PD-1 inhibitor may be administered simultaneously, at essentially the same time, or sequentially. SIK3 inhibitor and the PD-1 inhibitor need not be administered by means of the same vehicle. The SIK3 and the PD-1 inhibitor may be administered one or more times and the number of administrations of each component of the combination may be the same or different. In addition, the SIK3 inhibitor and the PD-1 inhibitor need not to be administered at the same site, not by the same route.

As used herein, the term "therapeutically effective" in context of a combination of the invention as used herein refers to an amount or dose of an SIK3 inhibitor together with the amount or dose of the PD-1 inhibitor that is sufficient to treat the disease, especially cancer. More preferably, the therapeutically effective in certain embodiments shall refer to an amount of PD-1 inhibitor and SIK3 inhibitor that elicits a synergistic therapeutic effect. The amount of the SIK3 inhibitor in a given therapeutically effective combination may be different for different individuals and different tumor types, and will be dependent upon the one or more additional agents or treatments included in the combination. The "therapeutically effective amount" is determined using procedures routinely employed by those of skill in the art such that an "improved therapeutic outcome" results. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidental with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Typically, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the patient to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

### Inhibitors of PD-1 and ligands thereof

As used herein the term "PD-1 inhibitor" refers to an agent which interferes with PD-1 activation or function. Examples of PD-1 inhibitors include anti-PD-1 antibodies (e.g. PD-1, PD-L1or PDL2 antibodies); small organic molecule PD-1 antagonists; and/or agents that bind to, or interfere with function of PD-1. Typically, the PD-1 inhibitor is an antibody or small organic molecule which binds to the PD-1 receptor or to its ligand PD-L1or PDL2.

In some embodiments, the PD-1 inhibitor is a small organic molecule. As used herein, the term "small organic molecule" refers to a molecule of size comparable to those organic molecules generally used in pharmaceuticals. The term excludes biological macromolecules (e.g.; proteins, nucleic acids, etc.); preferred small organic molecules range in size up to 2000 Da, and most preferably up to about 1000 Da.

Patent publications related to PD-1/PD-L1 antibodies include: WO2018/204303, CN108640992, WO2018/036472, CN107384933, WO2015/035606, CN107043425, CN106939050, CN106749663.

A non-exhaustive list of PD 1/PDLI antibodies comprises: Pembrolizumab (Keytruda^{®}), Nivolumab (Opdivo^{®}), BMS-936559 (MDX 1105), Cemiplimab (REGN2810), Cemiplimab- rwlc (LIBTAYO^{®}), Avelumab (MSB0010718C or Bavencio), Durvalumab (MEDI4736 or INFIMZI^{®}), Atezolizumab (MPDL3280A or Tecentriq^{®}), Spartalizumab (PDR 001), as well as combinations thereof.

Depending on the tumour to be treated in accordance with the present invention a specific PD-1 inhibitory compound may be selected for a combination with a SIK3 inhibitor. In this respect Atezolizumab or Avelumab or Durvalumab or Pembrolizumab or Nivolumab can be used for treating bladder cancer; Pembrolizumab or Nivolumab can be used for treating colorectal cancer or gastric cancer or head and neck cancer or Hodgkin's lymphoma; Nivolumab can be used for treating hepatocellular cancer; Nivolumab or Pembrolizumab or Ipilimumab and Nivolumab can be used for treating melanoma; Atezolizumab or Durvalumab or Pembrolizumab or Nivolumab can be used for treating lung cancer; Avelumab can be used for treating Merkel cell cancer; Pembrolizumab can be used for treating tissue-agnostic cancer.

As used herein, the term"antibody" is thus used to refer to any antibody-like molecule that has an antigen binding region, and this term includes antibody fragments that comprise an antigen binding domain such as Fab', Fab, F(ab')2, single domain antibodies (DABs), TandAbs dimer, Fv, scFv (single chain Fv), dsFv, ds-scFv, Fd, linear antibodies, minibodies, diabodies, bispecific antibody fragments, bibody, tribody (scFv-Fab fusions, bispecific or trispecific, respectively); sc-diabody; kappa(lamda) bodies (scFv-CL fusions); BiTE (Bispecific T-cell Engager, scFv-scFv tandems to attract T cells); DVD-Ig (dual variable domain antibody, bispecific format); SIP (small immunoprotein, a kind of minibody); SMIP ("small modular immunopharmaceutical" scFv-Fc dimer; DART (ds-stabilized diabody "Dual Affinity ReTargeting"); small antibody mimetics comprising one or more CDRs and the like. The techniques for preparing and using various antibody-based constructs and fragments are well known in the art (see Rabat et ak, 1991, specifically incorporated herein by reference). Diabodies, in particular, are further described in EP 404, 097 and WO 93/1 1 161; whereas linear antibodies are further described in Zapata et al. (1995). Antibodies can be fragmented using conventional techniques. For example, F(ab')2 fragments can be generated by treating the antibody with pepsin. The resulting F(ab')2 fragment can be treated to reduce disulfide bridges to produce Fab' fragments. Papain digestion can lead to the formation of Fab fragments. Fab, Fab' and F(ab')2, scFv, Fv, dsFv, Fd, dAbs, TandAbs, ds-scFv, dimers, minibodies, diabodies, bispecific antibody fragments and other fragments can also be synthesized by recombinant techniques or can be chemically synthesized. Techniques for producing antibody fragments are well known and described in the art. For example, each of Beckman et ak, 2006; Holliger & Hudson, 2005; Le Gall et ak, 2004; Reff & Heard, 2001; Reiter et ak, 1996; and Young et ak, 1995 further describe and enable the production of effective antibody fragments. In some embodiments, the antibody of the present invention is a single chain antibody. As used herein the term"single domain antibody" has its general meaning in the art and refers to the single heavy chain variable domain of antibodies of the type that can be found in Camelid mammals which are naturally devoid of light chains. Such single domain antibody are also"nanobody^{®}". For a general description of (single) domain antibodies, reference is also made to the prior art cited above, as well as to EP 0 368 684, Ward et ak (Nature 1989 Oct 12; 341 (6242): 544-6), Holt et ak, Trends Biotechnok, 2003, 21(11):484-490; and WO 06/030220, WO 06/003388. In some embodiments, the antibody is a humanized antibody. As used herein, "humanized" describes antibodies wherein some, most or all of the amino acids outside the CDR regions are replaced with corresponding amino acids derived from human immunoglobulin molecules. Methods of humanization include, but are not limited to, those described in U.S. Pat. Nos. 4,816,567, 5,225,539, 5,585,089, 5,693,761, 5,693,762 and 5,859,205, which are hereby incorporated by reference.

### SIK3 Inhibitor Compounds

"Salt-inducible kinase 3" or "SIK3" (synonyms QSK and KIAA0999) is a member of a subfamily of serine/threonine protein kinases including SIK1, SIK2, and SIK3 that belong to an AMP-activated protein kinase (AMPK) family. A SIK3 protein in context of the invention is, typically, a protein kinase. Pertinent information on the human SIK3 protein is accessible on UniProt: Q9Y2K2 (Entry version 138 of 15-Mar-2017) and a SIK3 protein in context of the invention has, preferably, an amino acid sequence shown in SIK3, Entry version 138 of 15-Mar-2017 or Entry version 144 of 28-Mar-2018, which sequences are incorporated herein by reference. SIK3 is a cytoplasmatic protein with serine/threonine kinase activity which is regulated through phosphorylation of a conserved threonine residue (position 163) in the T-loop of the kinase domain by the LKB1 complex; a phosphorylation which is reported as essential for catalytic activity of SIK3 (Lizcano, J. M. et al.; EMBO J. 23, 833-843 (2004)). For the purposes of the herein disclosed invention the term "phosphorylated SIK3" shall denote a SIK3 protein that is phosphorylated substantially as SIK3 protein can be (eg is) phosphorylated by LKB1, wherein preferably such phosphorylated SIK3 comprising a phosphor-threonine at amino acid position 163. A phosphorylated SIK3 in context of the invention is an SIK3 protein that is activated in its cell-biological context. At least four protein isoforms (SIK3-001 to SIK3-004) generated by alternative splicing of the SIK3 gene product are known. The human SIK3 gene is located at chromosomal position 11q23.3 (HGNC gene Symbol Acc: HGNC:29165), and is conserved in many species such as in chimpanzee, Rhesus monkey, dog, cow, mouse, rat, chicken, zebrafish, and frog. The term SIK3 in some embodiments of the invention may also pertain to variants of the human SIK3 protein having an amino acid sequence that is substantially identical to, or of at least 80%, preferably 85%, more preferably 90, 95, 96, 97, 98, 99, or 100% sequence identity to, the amino acid sequence of SIK3 as described above, as determined using, e.g., the "Blast 2 sequences" algorithm described by Tatusova & Madden 1999 (FEMS Microbiol Lett 174: 247-250), and which (preferably) retain biological activity identical or substantially identical to the respective reference SIK3 (eg to phosphorylate one or more class II (eg IIa) HDACs, such as HDAC4). Preferred variants of SIK3 protein comprise sequence variants thereof due to sequence polymorphism between and within populations of the respective species, as well as mutations compared to the wild-type sequence of SIK3 which are located in or in close proximity to the activity loop or activation loop (T-loop) of SIK3. A preferred variant of SIK3 protein is a SIK3 T163 mutation, such as a mutation affecting the activation of SIK3. In preferred embodiments a SIK3 protein of the invention is not a SIK1 (synonyms: SIK and SNF1LK) protein and/or is not a SIK2 (synonyms: QIK, KIAA0781 and SNF1LK2) protein. The amino acid sequence of human SIK1 (UniProt: P57059; entry version 168 of 15-Mar-2017) and human SIK2 (UniProt: Q9H0K1; entry version 153 of 15-Mar-2017) are incorporated herein by reference. The term SIK3 can mean, as applicable to the context (if not more specifically indicated), a SIK3 protein (such as one described above) or an mRNA molecule encoding such a SIK3 protein. The analogous meaning with respect of "SIK1" and "SIK2" is to be understood.

In **a first embodiment,** and as may be further described, defined, claimed or otherwise disclosed herein, the present invention provides as a SIK3 inhibitor **a compound** selected from the group consisting of a kinase inhibitor of the formula: 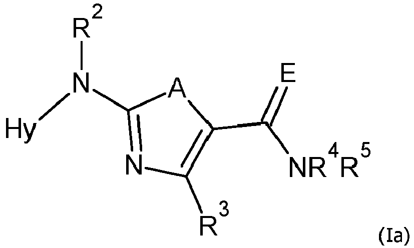 and solvates, salts, N-oxides, complexes, polymorphs, crystalline forms, racemic mixtures, diastereomers, enantiomers, tautomers, conformers, isotopically labeled forms, prodrugs, and combinations thereof; wherein:
**Hy** is a heteroaryl or heterocyclyl which is optionally substituted with one or more independently selected R^{1e}; **each R^{1e}** is independently selected from the group consisting of R^{1a}, R^{1b}, R^{1c} and R^{1d};
**each of R^{1a} and R^{1d}** is independently selected from the group consisting of H, alkyl, alkenyl, alkynyl, cycloalkyl, aryl, heterocyclyl, heteroaryl, halogen, -CN, azido, -NO₂, -OR¹¹, -N(R¹²)(R¹³), -N(R¹¹)(OR¹¹), -S(O)₀₋₂R¹¹, -S(O)₁₋₂OR¹¹, -OS(O)₁₋₂R¹¹ -OS(O)₁₋₂OR¹¹ -S(O)₁₋₂N(R¹²)(R¹³), -OS(O)₁₋₂N(R¹²)(R¹³), -N(R¹¹)S(O)₁₋₂R¹¹, -NR¹¹S(O)₁₋₂OR¹¹, -NR¹¹S(O)₁₋₂N(R¹²)(R¹³). -P(O)(OR¹¹)₂, -OP(O)(OR¹¹)₂, -C(=X)R¹¹, -C(=X)XR¹¹, -XC(=X)R¹¹, and -XC(=X)XR¹¹, wherein each of the alkyl, alkenyl, alkynyl, cycloalkyl, aryl, heterocyclyl, and heteroaryl groups is optionally substituted with one or more independently selected R³⁰;
**each of R^{1b} and R^{1c}** is independently selected from the group consisting of H, C₁₋₆ alkyl, C₂₋₆ alkenyl, C₂₋₆ alkynyl, C₃₋₇ cycloalkyl, C₆₋₁₀ aryl, 3- to 7-membered heteroaryl, 3- to 7-membered heterocyclyl, -O(CH₂)₀₋₂(C₃₋₇ cycloalkyl), -O(CH₂)₀₋₂(C₆₋₁₀ aryl), -O(CH₂)₀₋₂(3- to 7-membered heteroaryl), -O(CH₂)₀₋₂(3- to 7-membered heterocyclyl), -NH(CH₂)₀₋₂(C₃₋₇ cycloalkyl), -NH(CH₂)₀₋₂(C₆₋₁₀ aryl), -NH(CH₂)₀₋₂(3- to 7-membered heteroaryl), -NH(CH₂)₀₋₂(3- to 7-membered heterocyclyl), halogen, -CF₃, -CN, azido, -NO₂, -OH, -O(C₁₋₆ alkyl), -OCF₃, -S(C₁₋₆ alkyl), -NH₂, -NH(C₁₋₆ alkyl), -N(C₁₋₆ alkyl)₂, -NHS(O)₂(C₁₋₆ alkyl), -S(O)₂NH_{2-z}(C₁₋₆ alkyl)_{z}, -C(=O)(C₁₋₆ alkyl), -C(=O)OH, -C(=O)O(C₁₋₆ alkyl), -C(=O)NH_{2-z}(C₁₋₆ alkyl)_{z}, -NHC(=O)(C₁₋₆ alkyl), -NHC(=NH)NH_{z-2}(C₁₋₆ alkyl)_{z}, and -N(C₁₋₆ alkyl)C(=NH)NH_{2-z}(C₁₋₆ alkyl)_{z}, wherein z is 0, 1, or 2 and each of the C₁₋₆ alkyl, C₂₋₆ alkenyl, C₂₋₆ alkynyl, C₃₋₇ cycloalkyl, C₆₋₁₀ aryl, 3- to 7-membered heteroaryl, and 3- to 7-membered heterocyclyl groups is optionally substituted with one, two, or three moieties independently selected from the group consisting of -OH, methyl, ethyl, -OCH₃, -SCH₃, and -NH_{2-z}(CH₃)_{z};
**R²** is H;
**R³** is selected from the group consisting of H, alkyl, alkenyl, alkynyl, cycloalkyl, aryl, heterocyclyl, heteroaryl, halogen, -CN, azido, -NO₂, -OR¹¹, -N(R¹²)(R¹³), -N(R¹¹)(OR¹¹), -S(O)₀₋₂R¹¹ -S(O)₁₋₂OR¹¹ -OS(O)₁₋₂R¹¹ -OS(O)₁₋₂OR¹¹ -S(O)₁₋₂N(R¹²)(R¹³), -OS(O)₁₋₂N(R¹²)(R¹³), -N(R¹¹)S(O)₁₋₂R¹¹, -NR¹¹S(O)₁₋₂OR¹¹, -NR¹¹S(O)₁₋₂N(R¹²)(R¹³). -P(O)(OR¹¹)₂, -OP(O)(OR¹¹)₂, -C(=X)R¹¹, -C(=X)XR¹¹, -XC(=X)R¹¹, and -XC(=X)XR¹¹, wherein each of the alkyl, alkenyl, alkynyl, cycloalkyl, aryl, heterocyclyl, and heteroaryl groups is optionally substituted with one or more independently selected R³⁰;
**R⁴** is H;
**R⁵** is -L-R⁶;
L is selected from the group consisting of a bond, C₁₋₆ alkylene, C₂₋₆ alkenylene, C₂₋₆ alkynylene, and -(CH₂)ₘ-[Y-(CH₂)ₙ]ₒ-, wherein m is an integer between 1 and 6, n is an integer between 0 and 3, o is an integer between 1 and 3, wherein if n is 0 then o is 1; Y is independently selected from O, S, and -N(R¹³)-; and each of the C₁₋₆ alkylene, C₂₋₆ alkenylene, C₂₋₆ alkynylene, -(CH₂)ₘ-, and -(CH₂)ₙ- groups is optionally substituted with one or two independently selected R³⁰;
**R⁶** is a 5-membered monocyclic heteroaryl which contains at least one S ring atom and which is substituted with one or more independently selected R⁷;
**R⁷** is independently selected from the group consisting of alkyl, alkenyl, alkynyl, cycloalkyl, aryl, heterocyclyl, heteroaryl, halogen, -CN, azido, -NO₂, -OR¹¹, -N(R¹²)(R¹³), -N(R¹¹)(OR¹¹), -S(O)₀₋₂R¹¹ -S(O)₁₋₂OR¹¹, -OS(O)₁₋₂R¹¹ - OS(O)₁₋₂OR¹¹
   -S(O)₁₋₂N(R¹²)(R¹³), -OS(O)₁₋₂N(R¹²)(R¹³), -N(R¹¹)S(O)₁₋₂R¹¹, -NR¹¹S(O)₁₋₂OR¹¹, -NR¹¹S(O)₁₋₂N(R¹²)(R¹³). -P(O)(OR¹¹)₂, -OP(O)(OR¹¹)₂, -C(=X)R¹¹, -C(=X)XR¹¹, -XC(=X)R¹¹, and -XC(=X)XR¹¹, wherein each of the alkyl, alkenyl, alkynyl, cycloalkyl, aryl, heterocyclyl, and heteroaryl groups is optionally substituted with one or more independently selected R³⁰, wherein at least one of **R⁷** is F and/or at least one of **R⁷** is substituted with one or more F atoms;
**A** is selected from the group consisting of S, O, NR⁸, and C(R⁹)₂;
**R⁸** is selected from the group consisting of H, alkyl, alkenyl, alkynyl, cycloalkyl, aryl, heterocyclyl, and heteroaryl, wherein each of the alkyl, alkenyl, alkynyl, cycloalkyl, aryl, heterocyclyl, and heteroaryl groups is optionally substituted with one or more independently selected R³⁰;
**R⁹** is independently selected from the group consisting of alkyl, alkenyl, alkynyl, cycloalkyl, aryl, heterocyclyl, heteroaryl, halogen, -CN, azido, -NO₂, -OR¹¹, -N(R¹²)(R¹³), -S(O)₀₋₂R¹¹, -S(O)₁₋₂OR¹¹, -OS(O)₁₋₂R¹¹, -OS(O)₁₋₂OR¹¹ -S(O)₁₋₂N(R¹²)(R¹³), -OS(O)₁₋₂N(R¹²)(R¹³), -N(R¹¹)S(O)₁₋₂R¹¹, -NR¹¹S(O)₁₋₂OR¹¹, -NR11S(O)₁₋₂N(R¹²)(R¹³), -C(=X)R¹¹, -C(=X)XR¹¹, -XC(=X)R¹¹, and -XC(=X)XR¹¹, wherein each of the alkyl, alkenyl, alkynyl, cycloalkyl, aryl, heterocyclyl, and heteroaryl groups is optionally substituted with one or more independently selected R³⁰;
**X** is independently selected from the group consisting of O, S, and N(R¹⁴);
**E** is O or S;
**R¹¹** is independently selected from the group consisting of H, alkyl, alkenyl, alkynyl, cycloalkyl, aryl, heteroaryl, and heterocyclyl, wherein each of the alkyl, alkenyl, alkynyl, cycloalkyl, aryl, heteroaryl, and heterocyclyl groups is optionally substituted with one or more independently selected R³⁰;
**each of R¹² and R¹³** is independently selected from the group consisting of H, alkyl, alkenyl, alkynyl, cycloalkyl, aryl, heteroaryl, and heterocyclyl, or R¹² and R¹³ may join together with the nitrogen atom to which they are attached to form the group -N=CR¹⁵R¹⁶, wherein each of the alkyl, alkenyl, alkynyl, cycloalkyl, aryl, heteroaryl, and heterocyclyl groups is optionally substituted with one or more independently selected R³⁰;
**R¹⁴** is independently selected from the group consisting of H, alkyl, alkenyl, alkynyl, cycloalkyl, aryl, heteroaryl, heterocyclyl, and -OR¹¹, wherein each of the alkyl, alkenyl, alkynyl, cycloalkyl, aryl, heteroaryl, and heterocyclyl groups is optionally substituted with one or more independently selected R³⁰;
**each of R¹⁵ and R¹⁶** is independently selected from the group consisting of H, alkyl, alkenyl, alkynyl, cycloalkyl, aryl, heteroaryl, heterocyclyl, and -NH_{γ}R²⁰_{2-γ}, or R¹⁵ and R¹⁶ may join together with the atom to which they are attached to form a ring which is optionally substituted with one or more independently selected R³⁰, wherein each of the alkyl, alkenyl, alkynyl, cycloalkyl, aryl, heteroaryl, and heterocyclyl groups is optionally substituted with one or more independently selected R³⁰;
**y** is an integer from 0 to 2;
**R²⁰** is independently selected from the group consisting of alkyl, alkenyl, alkynyl, cycloalkyl, aryl, heteroaryl, and heterocyclyl, wherein each of the alkyl, alkenyl, alkynyl, cycloalkyl, aryl, heteroaryl, and heterocyclyl groups is optionally substituted with one or more independently selected R³⁰; and
**R³⁰** is a 1^{st} level substituent and is, in each case, independently selected from the group consisting of alkyl, alkenyl, alkynyl, aryl, heteroaryl, cycloalkyl, heterocyclyl, halogen, -CN, azido, -NO₂, -OR⁷¹, -N(R⁷²)(R⁷³), -S(O)₀₋₂R⁷¹, -S(O)₁₋₂OR⁷¹, -OS(O)₁₋₂R⁷¹, -OS(O)₁₋₂OR⁷¹, -S(O)₁₋₂N(R⁷²)(R⁷³), -OS(O)₁₋₂N(R⁷²)(R⁷³), -N(R⁷¹)S(O)₁₋₂R⁷¹, -NR⁷¹S(O)₁₋₂OR⁷¹, -NR⁷¹S(O)₁₋₂N(R⁷²)(R⁷³), -OP(O)(OR⁷¹)₂, -C(=X¹)R⁷¹, -C(=X¹)X¹R⁷, -X¹C(=X¹)R⁷¹, and -X¹C(=X¹)X¹R⁷¹, and/or any two R³⁰ which are bound to the same carbon atom of a cycloalkyl or heterocyclyl group may join together to form =X¹, wherein each of the alkyl, alkenyl, alkynyl, aryl, heteroaryl, cycloalkyl, and heterocyclyl groups being a 1^{st} level substituent is optionally substituted by one or more 2^{nd} level substituents, wherein said 2^{nd} level substituent is, in each case, independently selected from the group consisting of C₁₋₆ alkyl, C₂₋₆ alkenyl, C₂₋₆ alkynyl, 3- to 14-membered aryl, 3- to 14-membered heteroaryl, 3- to 14-membered cycloalkyl, 3- to 14-membered heterocyclyl, halogen, -CF₃, -CN, azido, -NO₂, -OR⁸¹, -N(R⁸²)(R⁸³), -S(O)₀₋₂R⁸¹, -S(O)₁₋₂OR⁸¹, -OS(O)₁₋₂R⁸¹, -OS(O)₁₋₂OR⁸¹, -S(O)₁₋₂N(R⁸²)(R⁸³), -OS(O)₁₋₂N(R⁸²)(R⁸³), -N(R⁸¹)S(O)₁₋₂R⁸¹, -NR⁸¹S(O)₁₋₂OR⁸¹, -NR⁸¹S(O)₁₋₂N(R⁸²)(R⁸³), -OP(O)(OR⁸¹)₂, -C(=X²)R⁸¹, -C(=X²)X²R⁸¹, -X²C(=X²)R⁸¹, and -X²C(=X²)X²R⁸¹, and/or any two 2^{nd} level substituents which are bound to the same carbon atom of a cycloalkyl or heterocyclyl group being a 1^{st} level substituent may join together to form =X², wherein each of the C₁₋₆ alkyl, C₂₋₆ alkenyl, C₂₋₆ alkynyl, 3- to 14-membered aryl, 3- to 14-membered heteroaryl, 3- to 14-membered cycloalkyl, 3- to 14-membered heterocyclyl groups being a 2^{nd} level substituent is optionally substituted with one or more 3^{rd} level substituents, wherein said 3^{rd} level substituent is, in each case, independently selected from the group consisting of C₁₋₃ alkyl, halogen, -CF₃, -CN, azido, -NO₂, -OH, -O(C₁₋₃ alkyl), -OCF₃, -S(C₁₋₃ alkyl), -NH₂, -NH(C₁₋₃ alkyl), -N(C₁₋₃ alkyl)₂, -NHS(O)₂(C₁₋₃ alkyl), -S(O)₂NH_{2-z}(C₁₋₃ alkyl)_{z}, -C(=O)OH, -C(=O)O(C₁₋₃ alkyl), -C(=O)NH_{2-z}(C₁₋₃ alkyl)_{z}, -NHC(=O)(C₁₋₃ alkyl), -NHC(=NH)NH_{z-2}(C₁₋₃ alkyl)_{z}, and -N(C₁₋₃ alkyl)C(=NH)NH_{2-z}(C₁₋₃ alkyl)_{z}, wherein each z is independently 0, 1, or 2 and each C₁₋₃ alkyl is independently methyl, ethyl, propyl or isopropyl, and/or any two 3^{rd} level substituents which are bound to the same carbon atom of a 3- to 14-membered cycloalkyl or heterocyclyl group being a 2^{nd} level substituent may join together to form =O, =S, =NH, or =N(C₁₋₃ alkyl);
wherein
**each of R⁷¹,R⁷², and R⁷³** is independently selected from the group consisting of H, C₁₋₆ alkyl, C₂₋₆ alkenyl, C₂₋₆ alkynyl, 3- to 7-membered cycloalkyl, 5- or 6-membered aryl, 5- or 6-membered heteroaryl, and 3- to 7-membered heterocyclyl, wherein each of the C₁₋₆ alkyl, C₂₋₆ alkenyl, C₂₋₆ alkynyl, 3- to 7-membered cycloalkyl, 5- or 6-membered aryl, 5- or 6-membered heteroaryl, and 3- to 7-membered heterocyclyl groups is optionally substituted with one, two or three substituents independently selected from the group consisting of C₁₋₃ alkyl, halogen, -CF₃, -CN, azido, -NO₂, -OH, -O(C₁₋₃ alkyl), -OCF₃, =O, -S(C₁₋₃ alkyl), -NH₂, -NH(C₁₋₃ alkyl), -N(C₁₋₃ alkyl)₂, -NHS(O)₂(C₁₋₃ alkyl), -S(O)₂NH_{2-z}(C₁₋₃ alkyl)_{z}, -C(=O)(C₁₋₃ alkyl), -C(=O)OH, -C(=O)O(C₁₋₃ alkyl), -C(=O)NH_{2-z}(C₁₋₃ alkyl)_{z}, -NHC(=O)(C₁₋₃ alkyl), -NHC(=NH)NH_{z-2}(C₁₋₃ alkyl)_{z}, and -N(C₁₋₃ alkyl)C(=NH)NH_{2-z}(C₁₋₃ alkyl)_{z}, wherein each z is independently 0, 1, or 2 and each C₁₋₃ alkyl is independently methyl, ethyl, propyl or isopropyl;
**each of R⁸¹, R⁸², and R⁸³** is independently selected from the group consisting of H, C₁₋₄ alkyl, C₂₋₄ alkenyl, C₂₋₄ alkynyl, 3- to 6-membered cycloalkyl, 5- or 6-membered aryl, 5- or 6-membered heteroaryl, and 3- to 6-membered heterocyclyl, wherein each of the C₁₋₄ alkyl, C₂₋₄ alkenyl, C₂₋₄ alkynyl, 3- to 6-membered cycloalkyl, 5- or 6-membered aryl, 5- or 6-membered heteroaryl, and 3- to 6-membered heterocyclyl groups is optionally substituted with one, two or three substituents independently selected from the group consisting of C₁₋₃ alkyl, halogen, -CF₃, -CN, azido, -NO₂, -OH, -O(C₁₋₃ alkyl), -OCF₃, =O, -S(C₁₋₃ alkyl), -NH₂, -NH(C₁₋₃ alkyl), -N(C₁₋₃ alkyl)₂, -NHS(O)₂(C₁₋₃ alkyl), -S(O)₂NH_{2-z}(C₁₋₃ alkyl)_{z}, -C(=O)(C₁₋₃ alkyl), -C(=O)OH, -C(=O)O(C₁₋₃ alkyl), -C(=O)NH_{2-z}(C₁₋₃ alkyl)_{z}, -NHC(=O)(C₁₋₃ alkyl), -NHC(=NH)NH_{z-2}(C₁₋₃ alkyl)_{z}, and -N(C₁₋₃ alkyl)C(=NH)NH_{2-z}(C₁₋₃ alkyl)_{z}, wherein each z is independently 0,1, or 2 and
**each C₁₋₃** alkyl is independently methyl, ethyl, propyl or isopropyl; and
**each of X¹ and X²** is independently selected from O, S, and N(R⁸⁴), wherein R⁸⁴ is H or C₁₋₃ alkyl.

Such compounds are which are kinase inhibitors are in detail defined and exemplified in international patent publication WO 2021/214117 which shall for the purpose of the definition of SIK3 inhibitors of the invention be incorporated herein in its entirety.

A selection of SIK3 inhibitor compounds, including those which have been synthesized and tested, within the scope of, or for use within the methods of, the disclosure of WO 2021/214117 - is listed in the following Table A.

**Table A: Kinase inhibitors of formula (Ia).**

| Compound Number | Structure | Name |
|---|---|---|
| E1 | 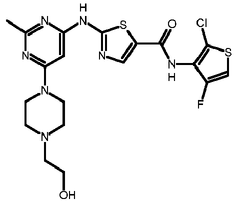 | *N*-(2-chloro-4-fluorothiophen-3-yl)-2-((6-(4-(2-hydroxyethyl)piperazin-1-yl)-2-methylpyrimidin-4-yl)amino)thiazole-5-carboxamide |
| E2 | 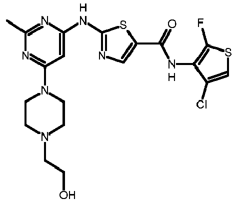 | *N*-(4-chloro-2-fluorothiophen-3-yl)-2-((6-(4-(2-hydroxyethyl)piperazin-1-yl)-2-methylpyrimidin-4-yl)amino)thiazole-5-carboxamide |
| E3 | 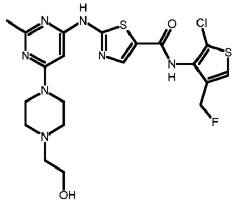 | *N*-(2-chloro-4-(fluoromethyl)thiophen-3-yl)-2-((6-(4-(2-hydroxyethyl)piperazin-1-yl)-2-methylpyrimidin-4-yl)amino)thiazole-5-carboxamide |
| E4 | 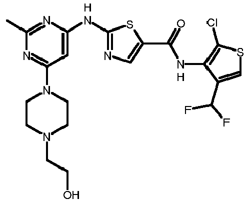 | *N*-(2-chloro-4-(difluoromethyl)thiophen-3-yl)-2-((6-(4-(2-hydroxyethyl)piperazin-1-yl)-2-methylpyrimidin-4-yl)amino)thiazole-5-carboxamide |
| E5 | 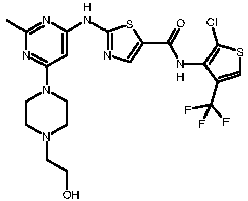 | *N*-(2-chloro-4-(trifluoromethyl)thiophen-3-yl)-2-((6-(4-(2-hydroxyethyl)piperazin-1-yl)-2-methylpyrimidin-4-yl)amino)thiazole-5-carboxamide |
| E6 | 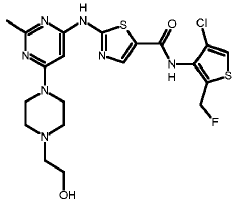 | *N*-(4-chloro-2-(fluoromethyl)thiophen-3-yl)-2-((6-(4-(2-hydroxyethyl)piperazin-1-yl)-2-methylpyrimidin-4-yl)amino)thiazole-5-carboxamide |
| E7 | 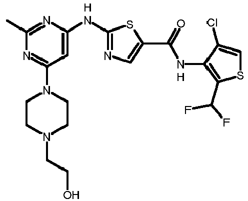 | *N*-(4-chloro-2-(difluoromethyl)thiophen-3-yl)-2-((6-(4-(2-hydroxyethyl)piperazin-1-yl)-2-methylpyrimidin-4-yl)amino)thiazole-5-carboxamide |
| E8 | 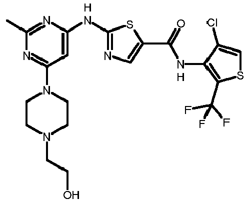 | *N*-(4-chloro-2-(trifluoromethyl)thiophen-3-yl)-2-((6-(4-(2-hydroxyethyl)piperazin-1-yl)-2-methylpyrimidin-4-yl)amino)thiazole-5-carboxamide |
| E9 | 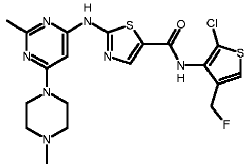 | *N*-(2-chloro-4-(fluoromethyl)thiophen-3-yl)-2-((2-methyl-6-(4-methylpiperazin-1-yl)pyrimidin-4-yl)amino)thiazole-5-carboxamide |
| E10 | 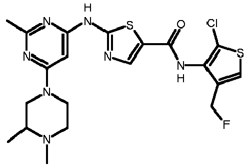 | *N*-(2-chloro-4-(fluoromethyl)thiophen-3-yl)-2-((6-(3,4-dimethylpiperazin-1-yl)-2-methylpyrimidin-4-yl)amino)thiazole-5-carboxamide |
| E11 | 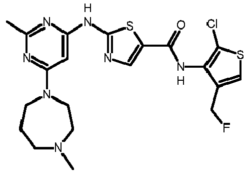 | *N*-(2-chloro-4-(fluoromethyl)thiophen-3-yl)-2-((2-methyl-6-(4-methyl-1,4-diazepan-1-yl)pyrimidin-4-yl)amino)thiazole-5-carboxamide |
| E12 | 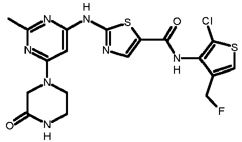 | *N*-(2-chloro-4-(fluoromethyl)thiophen-3-yl)-2-((2-methyl-6-(3-oxopiperazin-1-yl)pyrimidin-4-yl)amino)thiazole-5-carboxamide |
| E13 | 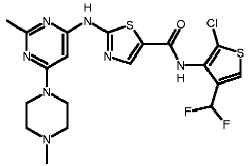 | *N*-(2-chloro-4-(difluoromethyl)thiophen-3-yl)-2-((6-(3,4-dimethylpiperazin-1-yl)-2-methyl pyrimidin-4-yl)amino)thiazole-5-carboxamide |
| E14 | 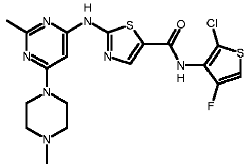 | *N*-(2-chloro-4-fluorothiophen-3-yl)-2-((2-methyl-6-(4-methylpiperazin-1-yl)pyrimidin-4-yl)amino)thiazole-5-carboxamide |
| E15 | 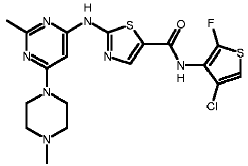 | *N*-(4-chloro-2-fluorothiophen-3-yl)-2-((2-methyl-6-(4-methylpiperazin-1-yl)pyrimidin-4-yl)amino)thiazole-5-carboxamide |
| E16 | 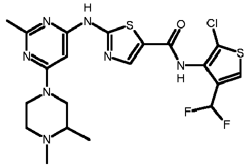 | *N*-(2-chloro-4-(difluoromethyl)thiophen-3-yl)-2-((2-methyl-6-(3,4-dimethyl piperazin-1-yl)pyrimidin-4-yl)amino)thiazole-5-carboxamide |

In particular embodiments, the compound of the invention is selected from the group consisting of E4, E9, E10, and E16; and also their solvates, salts, N-oxides, complexes, polymorphs, crystalline forms, racemic mixtures, diastereomers, enantiomers, tautomers, conformers, isotopically labelled forms, prodrugs, and combinations thereof.

In certain embodiments, the compound of the invention is E9, or a solvate, salt, N-oxide, complex, polymorph, crystalline form, tautomer, conformer, isotopically labelled form, prodrug, or combination thereof.

In another certain embodiment, the compound of the invention is E4, or a solvate, salt, N-oxide, complex, polymorph, crystalline form, tautomer, conformer, isotopically labelled form, prodrug, or combination thereof.

In another certain embodiment, the compound of the invention is E10, or a solvate, salt, N-oxide, complex, polymorph, crystalline form, tautomer, conformer, isotopically labelled form, prodrug, or combination thereof.

In another certain embodiment, the compound of the invention is E16, or a solvate, salt, N-oxide, complex, polymorph, crystalline form, racemic mixture, diastereomer, enantiomer, tautomer, conformer, isotopically labelled form, prodrug, or combination thereof.

In certain embodiments, the invention may relate to a solvate, salt, N-oxide, complex, racemic mixture, diastereomer, enantiomer, tautomer, conformer, isotopically labeled form, prodrug, or combination thereof, of any of the compounds of the invention or of any of the compounds used in the invention; such as a solvate, salt, complex, racemic mixture, diastereomer, enantiomer, tautomer, conformer, isotopically labeled form, or combination thereof, of such compound.

The SIK3 inhibitor compounds of the invention (and/or the compounds used in the invention) may be in a prodrug form. Prodrugs of the compounds of the invention (or of the compounds used in the invention) are those compounds that upon administration to an individual undergo chemical conversion under physiological conditions to provide the compounds of the invention. Additionally, prodrugs can be converted to the compounds of the invention (or of the compounds used in the invention) by chemical or biochemical methods in an *ex vivo* environment. For example, prodrugs can be slowly converted to the compounds of the invention (or to the compounds used in the invention) when, for example, placed in a transdermal patch reservoir with a suitable enzyme or chemical reagent. Exemplary prodrugs are esters (using an alcohol or a carboxy group contained in the kinase inhibitor of the invention (or in the compounds used in the invention)) or amides (using an amino or a carboxy group contained in the kinase inhibitor of the invention (or in the compounds used in the invention)) which are hydrolyzable *in vivo.* Specifically, any amino group which is contained in the kinase inhibitor of the invention (or in the compounds used in the invention) and which bears at least one hydrogen atom can be converted into a prodrug form.

In another embodiment, the compounds of the invention exhibit one or more pharmacological properties that are different to those of dasatinib, of compound B3 (WO 2018/193084), and/or of compound C7 (PCT/EP2019/078751).

Other than the above small molecular kinase inhibitors, the invention shall also include other strategies to interfere with the cellular function of SIK3. Other compounds are for example known from WO/2021/219731, WO/2019/202160 and WO/2018/193084.

A compound being an "inhibitor of SIK3" (or "SIK3 inhibitor") is any moiety that inhibits SIK3, which can mean inhibition of the activity of SIK3, especially of protein of SIK3, and in particular of phosphorylated SIK3. A SIK3 inhibitor may impair (eg, induces a decrease or reduction in) the efficiency, effectiveness, amount or rate of one or more activities of SIK3, such as one or more of those activities described herein, for example, the activity of SIK3 to phosphorylate class II (eg Ila) HDACs (eg HDAC4) and/or to sensitise a cell involved with a proliferative disorder to a cell-mediated immune response. Such a SIK3 inhibiting moiety can act directly, for example, by binding to SIK3 and decreasing the amount or rate of one or more of the properties of SIK3 such as its function, in particular its ability to act as a kinase (eg to phosphorylate HDAC4), for example by reducing the activity of phosphorylated SIK3 in the cell.

In certain embodiments of these combination aspects, the inhibitor or antagonist of said SIK3 kinase or variant is, preferably, an inhibitory or antagonistic small molecule as described herein. In other embodiments of these combination aspects, said inhibitor or antagonist of said SIK3 or variant, is a compound selected from a polypeptide, peptide, glycoprotein, a peptidomimetic, an antibody or antibody-like molecule; a nucleic acid such as a DNA or RNA, for example an antisense DNA or RNA, a ribozyme, an RNA or DNA aptamer, siRNA, shRNA and the like, including variants or derivatives thereof such as a peptide nucleic acid (PNA); a targeted gene editing construct, such as a CRISPR/Cas9 construct, a carbohydrate such as a polysaccharide or oligosaccharide and the like, including variants or derivatives thereof; a lipid such as a fatty acid and the like, including variants or derivatives thereof; or a small organic molecules including but not limited to small molecule ligands, small cell-permeable molecules, and peptidomimetic compounds.

### Pharmaceutical compositions

The compounds described in the present invention (in particular those SIK3 inhibitor compounds above particularly those given in Table A) or the compounds used in the present invention are preferably administered to a patient in need thereof via a pharmaceutical composition. Thus, in **a second aspect,** the present invention provides **a pharmaceutical composition** comprising a kinase inhibitor as specified above under the heading "Compounds" (e.g., a SIK3 kinase inhibitor having the general formula (Ia), complex, polymorph, crystalline form, racemic mixture, diastereomer, enantiomer, tautomer, conformer, isotopically labeled form, prodrug, or combination thereof and optionally one or more pharmaceutically acceptable excipients.

Thus, in one embodiment the pharmaceutical composition comprises a kinase inhibitor as specified above under the heading "Compounds" (in particular a compound of the first aspect of the invention) and one or more pharmaceutically acceptable excipients. Furthermore, the pharmaceutical composition may further comprise one or more additional therapeutic agents. Thus, in particular embodiments, the pharmaceutical composition comprises (i) a kinase inhibitor as specified above under the heading "Compounds" (in particular a compound of the first aspect of the invention) and one or more additional therapeutic agents; or (ii) a kinase inhibitor as specified above under the heading "Compounds" (in particular a compound of the first aspect of the invention), one or more additional therapeutic agents, and one or more pharmaceutically acceptable excipients.

The term "pharmaceutically acceptable" refers to the non-toxicity of a material which does not interact with the (e.g., therapeutic) action of the active component (e.g., a kinase inhibitor of the invention (or a compound used in the invention), either alone or in combination with one or more additional therapeutic agents) of the pharmaceutical composition.

The pharmaceutical composition may be administered to an individual by any route, such as enterally or parenterally.

The expressions "enteral administration" and "administered enterally" as used herein mean that the drug administered is taken up by the stomach and/or the intestine. Examples of enteral administration include oral and rectal administration. The expressions "parenteral administration" and "administered parenterally" as used herein mean modes of administration other than enteral administration, usually by injection or topical application, and include, without limitation, intravenous, intramuscular, intraarterial, intrathecal, intracapsular, intraosseous, intraorbital, intracardiac, intradermal, intraperitoneal, transtracheal, subcutaneous, subcuticular, intraarticular, subcapsular, intracerebral, intracerebroventricular, subarachnoid, intraspinal, epidural and intrasternal administration (such as by injection and/or infusion) as well as topical administration (e.g., epicutaneous, inhalational, or through mucous membranes (such as buccal, sublingual or vaginal)).

Dosage forms for topical and/or transdermal administration of a compound described herein may include ointments, pastes, creams, lotions, gels, powders, solutions, sprays, inhalants, and/or patches. Generally, the active ingredient is admixed under sterile conditions with a pharmaceutically acceptable excipient such as one or more pharmaceutical carriers) and/or any needed preservatives and/or buffers as can be required. Additionally, the present disclosure contemplates the use of transdermal patches, which often have the added advantage of providing controlled delivery of an active ingredient to the body. Such dosage forms can be prepared, for example, by dissolving and/or dispensing the active ingredient in the proper medium. Alternatively, or additionally, the rate can be controlled by either providing a rate controlling membrane and/or by dispersing the active ingredient in a polymer matrix and/or gel.

Suitable devices for use in delivering intradermal pharmaceutical compositions described herein include short needle devices. Intradermal compositions can be administered by devices which limit the effective penetration length of a needle into the skin. Alternatively, or additionally, conventional syringes can be used in the classical mantoux method of intradermal administration. Jet injection devices which deliver liquid formulations to the dermis via a liquid jet injector and/or via a needle which pierces the stratum corneum and produces a jet which reaches the dermis are suitable. Ballistic powder/particle delivery devices which use compressed gas to accelerate the compound in powder form through the outer layers of the skin to the dermis are suitable.

Formulations suitable for topical administration include, but are not limited to, liquid and/or semi-liquid preparations such as liniments, lotions, oil-in-water and/or water-in-oil emulsions such as creams, ointments, and/or pastes, and/or solutions and/or suspensions. Topically administrable formulations may, for example, comprise from about 1% to about 10% (w/w) active ingredient, although the concentration of the active ingredient can be as high as the solubility limit of the active ingredient in the solvent. Formulations for topical administration may further comprise one or more of the additional ingredients described herein.

The compounds of the present invention (or the compounds used in the present invention) are generally applied in "pharmaceutically acceptable amounts" and in "pharmaceutically acceptable preparations". Such compositions may contain salts, buffers, preserving agents, carriers and optionally other therapeutic agents. "Pharmaceutically acceptable salts" comprise, for example, acid addition salts which may, for example, be formed by mixing a solution of compounds with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compound carries an acidic moiety, suitable pharmaceutically acceptable salts thereof may include alkali metal salts (e.g., sodium or potassium salts); alkaline earth metal salts (e.g., calcium or magnesium salts); and salts formed with suitable organic ligands (e.g., ammonium, quaternary ammonium and amine cations formed using counteranions such as halide, hydroxide, carboxylate, sulfate, phosphate, nitrate, alkyl sulfonate and aryl sulfonate). Illustrative examples of pharmaceutically acceptable salts include, but are not limited to, acetate, adipate, alginate, arginate, ascorbate, aspartate, benzenesulfonate, benzoate, bicarbonate, bisulfate, bitartrate, borate, bromide, butyrate, calcium edetate, camphorate, camphorsulfonate, camsylate, carbonate, chloride, citrate, clavulanate, cyclopentanepropionate, digluconate, dihydrochloride, dodecylsulfate, edetate, edisylate, estolate, esylate, ethanesulfonate, formate, fumarate, galactate, galacturonate, gluceptate, glucoheptonate, gluconate, glutamate, glycerophosphate, glycolylarsanilate, hemisulfate, heptanoate, hexanoate, hexylresorcinate, hydrabamine, hydrobromide, hydrochloride, hydroiodide, 2-hydroxy-ethanesulfonate, hydroxynaphthoate, iodide, isobutyrate, isothionate, lactate, lactobionate, laurate, lauryl sulfate, malate, maleate, malonate, mandelate, mesylate, methanesulfonate, methylsulfate, mucate, 2-naphthalenesulfonate, napsylate, nicotinate, nitrate, N-methylglucamine ammonium salt, oleate, oxalate, pamoate (embonate), palmitate, pantothenate, pectinate, persulfate, 3-phenylpropionate, phosphate/diphosphate, phthalate, picrate, pivalate, polygalacturonate, propionate, salicylate, stearate, sulfate, suberate, succinate, tannate, tartrate, teoclate, tosylate, triethiodide, undecanoate, valerate, and the like (see, for example, Berge et al., "Pharmaceutical Salts", J. Pharm. Sci., 66, pp. 1-19 (1977)).

The term "excipient" when used herein is intended to indicate all substances in a pharmaceutical composition which are not active ingredients (e.g., which are therapeutically inactive ingredients that do not exhibit any therapeutic effect in the amount/concentration used), such as, e.g., carriers, binders, lubricants, thickeners, surface active agents, preservatives, stabilizers, emulsifiers, buffers, flavoring agents, colorants, or antioxidants.

The compositions described in the present invention may comprise a pharmaceutically acceptable carrier. As used herein, "pharmaceutically acceptable carrier" includes any and all solvents, dispersion media, coatings, isotonic and absorption delaying agents, and the like that are physiologically compatible. The "pharmaceutically acceptable carrier" may be in the form of a solid, semisolid, liquid, or combinations thereof. Preferably, the carrier is suitable for enteral (such as oral) or parenteral administration (such as intravenous, intramuscular, subcutaneous, spinal or epidermal administration (e.g., by injection or infusion)). Depending on the route of administration, the active compound, e.g., the compound of the present invention (or the compound used in the present invention), either alone or in combination with one or more additional therapeutic agents, may be coated in a material to protect the active compound(s) from the action of acids and other natural conditions that may inactivate the active compound.

Examples of suitable aqueous and non-aqueous carriers which may be employed in the pharmaceutical compositions according to the present invention include water (e.g., water for injection), ethanol, polyols (such as glycerol, propylene glycol, polyethylene glycol, and the like), aqueous solutions of a salt, carbohydrate, sugar alcohol, or an amino acid (such as saline or an aqueous amino acid solution), and suitable mixtures and/or buffered forms thereof, vegetable oils (such as olive oil), and injectable organic esters (such as ethyl oleate). Proper fluidity can be maintained, for example, by the use of coating materials, such as lecithin, by the maintenance of the required particle size in the case of dispersions, and by the use of surfactants.

Pharmaceutically acceptable carriers include sterile aqueous solutions or dispersions and sterile powders for the extemporaneous preparation of sterile injectable solutions or dispersions. The use of such media and agents for pharmaceutically active compounds is known in the art. Except insofar as any conventional media or agent is incompatible with the active compound, use thereof in the pharmaceutical compositions according to the present invention is contemplated.

The pharmaceutical composition described herein may comprise, in addition to the kinase inhibitor of the invention and the PD-1 inhibitor (and/or the compound or composition used in the invention), at least one, e.g., 1, 2, 3, 4, 5, 6, 7 or 8, additional therapeutic agents. According to the present teaching, the at least one additional therapeutic agent may be formulated together with the kinase inhibitor of the invention (and/or with the compound used in the invention) in a single pharmaceutical composition. Alternatively, the pharmaceutical composition may be structured as kit of parts, wherein the kinase inhibitor of the invention (or the compound used in the invention) is provided in a first formulation and the at least one additional therapeutic agent is provided in a second formulation, i.e., a second pharmaceutical composition. The first and the second pharmaceutical compositions may be combined prior to use. In other words, before administering the pharmaceutical composition, a formulation comprising the additional therapeutic agent may be added to the first pharmaceutical composition comprising the kinase inhibitor of the invention (or the compound used in the invention). Alternatively, the present teaching envisages administering the kinase inhibitor of the invention (or the compound used in the invention) formulated in a first pharmaceutical composition and administering the at least one additional therapeutic agent formulated in a second pharmaceutical composition. The pharmaceutical compositions may be administered concomitantly or in succession. For example, the first pharmaceutical composition may be administered at a first point in time and the second pharmaceutical composition may be administered at a second point in time, wherein the points in time may be separated by, for example, 0, or up to 1, 2, 3, 4, 5 or 10 min, up to 1, 2, 3, 4, 5 or 10 hours, up to 1, 2, 3, 4, 5 or 10 days, up to 1, 2, 3, 4, 5 or 10 weeks, up to 1, 2, 3, 4, 5 or 10 months or up to 1, 2, 3, 4, 5 or 10 years.

The compositions may also contain adjuvants such as preservatives, stabilizers, wetting agents, emulsifying agents, pH buffering agents, and dispersing agents. Prevention of the presence of microorganisms may be ensured by sterilization procedures and/or by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Regardless of the route of administration selected, the active compounds, which may be used in a suitable hydrated form, and/or the pharmaceutical compositions according to the present invention, are formulated into pharmaceutically acceptable dosage forms by conventional methods known to those of skill in the art (cf., e.g., Remington, "The Science and Practice of Pharmacy" edited by Allen, Loyd V., Jr., 22nd edition, Pharmaceutical Sciences, September 2012; Ansel et al., "Pharmaceutical Dosage Forms and Drug Delivery Systems", 7th edition, Lippincott Williams & Wilkins Publishers, 1999).

A pharmaceutical composition can be administered by a variety of methods known in the art. As will be appreciated by the skilled artisan, the route and/or mode of administration will vary depending upon the desired results. The pharmaceutical compositions containing one or more active compounds can be prepared with carriers that will protect the one or more active compounds against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Methods for the preparation of such compositions are generally known to those skilled in the art. See, e.g., Sustained and Controlled Release Drug Delivery Systems, J. R. Robinson, ed., Marcel Dekker, Inc., New York, 1978.

To administer a compound of the present invention (or a compound used in the present invention) by certain routes of administration, it may be necessary to coat the compound with, or co-administer the compound with, a material to prevent its inactivation. For example, the compound may be administered to an individual in an appropriate carrier, for example, liposomes, or a diluent. Pharmaceutically acceptable diluents include saline and aqueous buffer solutions. Liposomes include water-in-oil-in-water CGF emulsions as well as conventional liposomes (Strejan et al., J. Neuroimmunol. 7: 27(1984)).

Pharmaceutical compositions typically are sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin.

An injectable composition should be sterile and fluid to the extent that the composition is deliverable by syringe. In addition to water, the carrier can be an isotonic buffered saline solution, ethanol, polyol (for example, glycerol, propylene glycol, and liquid polyethylene glycol, and the like), and suitable mixtures thereof. Sterile injectable solutions can be prepared by incorporating the active compound in the required amount in an appropriate solvent with one or a combination of ingredients enumerated above, as required, followed by sterilization microfiltration.

Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the individuals to be treated; each unit contains a predetermined quantity of active compound calculated to produce the desired therapeutic effect in association with the required pharmaceutical carrier. The specification for the dosage unit forms used according to the present invention are dictated by and directly dependent on (a) the unique characteristics of the active compound and the particular therapeutic effect to be achieved, and (b) the limitations inherent in the art of compounding such an active compound for the treatment of sensitivity in individuals.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

For the therapeutic/pharmaceutical formulations, compositions according to the present invention include those suitable for enteral administration (such as oral or rectal) or parenteral administration (such as nasal, topical (including vaginal, buccal and sublingual)). The compositions may conveniently be presented in unit dosage form and may be prepared by any methods known in the art of pharmacy. The amount of active ingredient (in particular, the amount of a compound according to the present invention) which can be combined with a carrier material to produce a pharmaceutical composition (such as a single dosage form) will vary depending upon the individual being treated, and the particular mode of administration. The amount of active ingredient which can be combined with a carrier material to produce a single dosage form will generally be that amount of the composition which produces a therapeutic effect.

Generally, out of 100% (for the pharmaceutical formulations/compositions), the amount of active ingredient (in particular, the amount of the compound according to the present invention (or of the compound used in the present invention), optionally together with other therapeutically active agents, if present in the pharmaceutical formulations/compositions) will range from about 0.01% to about 99%, preferably from about 0.1% to about 70%, most preferably from about 1% to about 30%, wherein the reminder is preferably composed of the one or more pharmaceutically acceptable excipients.

The amount of active ingredient, e.g., a compound according to the present invention (or a compound used in the present invention), in a unit dosage form and/or when administered to an individual or used in therapy, may range from about 0.1 mg to about 1000 mg (for example, from about 1 mg to about 500 mg, such as from about 10 mg to about 200 mg) per unit, administration or therapy. In certain embodiments, a suitable amount of such active ingredient may be calculated using the mass or body surface area of the individual, including amounts of between about 1 mg/kg and 10 mg/kg (such as between about 2 mg/kg and 5 mg/kg), or between about 1 mg/m² and about 400 mg/m² (such as between about 3 mg/m² and about 350 mg/m² or between about 10 mg/m² and about 200 mg/m²).

Actual dosage levels of the active ingredients in the pharmaceutical compositions according to the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

A physician or veterinarian having ordinary skill in the art can readily determine and prescribe the (e.g., therapeutically) effective amount of the pharmaceutical composition required. For example, the physician or veterinarian could start with doses of the compounds according to the present invention (or of the compounds used in the present invention) at levels lower than that required in order to achieve the desired therapeutic effect and gradually increase the dosage until the desired effect is achieved. In general, a suitable daily dose of a composition according to the present invention will be that amount of the compound which is the lowest dose effective to produce a therapeutic effect. Such an effective dose will generally depend upon the factors described above. It is preferred that administration be oral, intravenous, intramuscular, intraperitoneal, or subcutaneous, preferably administered proximal to the site of the target. If desired, the (e.g., therapeutically) effective daily dose of a pharmaceutical composition may be administered as two, three, four, five, six or more sub-doses administered separately at appropriate intervals throughout the day, optionally, in unit dosage forms. While it is possible for a compound according to the present invention (or for the compound used in the present invention) to be administered alone, it is preferable to administer the compound as a pharmaceutical formulation/composition.

For oral administration, the pharmaceutical composition according to the present invention can take the form of, for example, tablets or capsules prepared by conventional means with pharmaceutical acceptable excipients such as binding agents (e.g., pregelatinised maize starch, polyvinylpyrrolidone, hydroxypropyl methylcellulose), fillers (e.g., lactose, microcrystalline cellulose, calcium hydrogen phosphate), lubricants (e.g., magnesium stearate, talc, silica), disintegrants (e.g., potato starch, sodium starch glycolate), or wetting agents (e.g., sodium lauryl sulphate). Liquid preparations for oral administration can be in the form of, for example, solutions, syrups, or suspensions, or can be presented as a dry product for constitution with water or other suitable vehicle before use. Such liquid preparation can be prepared by conventional means with pharmaceutically acceptable additives such as suspending agents (e.g., sorbitol, syrup, cellulose derivatives, hydrogenated edible fats), emulsifying agents (e.g., lecithin, acacia), non-aqueous vehicles (e.g., almond oil, oily esters, ethyl alcohol, fractionated vegetable oils), preservatives (e.g., methyl or propyl-p-hydroxycarbonates, sorbic acids). The preparations can also contain buffer salts, flavouring, coloring and sweetening agents as deemed appropriate. Preparations for oral administration can be suitably formulated to give controlled release of the pharmaceutical composition of the invention.

In one embodiment, the compound is orally administered in a concentration of, for example, at most 100 mg/kg body weight (such as at most 50 mg/kg body weight, at most 40 mg/kg body weight, at most 30 mg/kg body weight, at most 20 mg/kg body weight, at most 10 mg/kg body weight, at most 5 mg/kg body weight, at most 4 mg/kg body weight, at most 3 mg/kg body weight, at most 2 mg/kg body weight, at most 1 mg/kg body weight).

In one embodiment, the compound is parenterally administered (e.g., intravenously, intramuscularly, or subcutaneously), in a concentration of, for example, at most 10 mg/kg body weight (such as at most 5 mg/kg body weight, at most 4 mg/kg body weight, at most 3 mg/kg body weight, at most 2 mg/kg body weight, at most 1 mg/kg body weight, at most 0.5 mg/kg body weight, at most 0.4 mg/kg body weight, at most 0.3 mg/kg body weight, at most 0.2 mg/kg body weight, at most 0.1 mg/kg body weight).

The pharmaceutical composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc.

For administration by inhalation, the pharmaceutical composition according to the present invention is conveniently delivered in the form of an aerosol spray presentation from a pressurized pack or a nebulizer, with the use of a suitable propellant (e.g., dichlorodifluoromethane, trichlorofluoromethane, dichlorotetrafluoroethane, carbon dioxide, nitrogen, or other suitable gas). In the case of a pressurized aerosol, the dosage unit can be determined by providing a valve to deliver a metered amount. Capsules and cartridges of, for example, gelatine, for use in an inhaler or insufflator can be formulated containing a powder mix of the pharmaceutical composition according to the present invention and a suitable powder base such as lactose or starch.

The pharmaceutical composition according to the present invention can be formulated for parenteral administration by injection, for example, by bolus injection or continuous infusion. In one embodiment, the compounds or compositions according to the present invention may be administered by slow continuous infusion over a long period, such as more than 24 hours, in order to reduce toxic side effects. The administration may also be performed by continuous infusion over a period of from 2 to 24 hours, such as of from 2 to 12 hours. Such regimen may be repeated one or more times as necessary, for example, after 6 months or 12 months.

In yet another embodiment, the compounds or compositions according to the present invention are administered by maintenance therapy, such as, e.g., once a week for a period of 6 months or more.

Formulations for injection can be presented in units dosage form (e.g., in phial, in multi-dose container), and with an added preservative. The pharmaceutical composition according to the present invention can take such forms as suspensions, solutions or emulsions in oily or aqueous vehicles, and can contain formulatory agents such as suspending, stabilizing, or dispersing agents. Alternatively, the agent can be in powder form for constitution with a suitable vehicle (e.g., sterile pyrogen-free water) before use. Typically, compositions for intravenous administration are solutions in sterile isotonic aqueous buffer. Where necessary, the composition can also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients can be mixed prior to administration.

Compositions according to the present invention which are suitable for vaginal administration also include pessaries, tampons, creams, gels, pastes, foams or spray formulations containing such carriers as are known in the art to be appropriate. Dosage forms for the topical or transdermal administration of compositions according to the present invention include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required.

Therapeutic/pharmaceutical compositions can be administered with medical devices known in the art. For example, in a preferred embodiment, a therapeutic/pharmaceutical composition according to the present invention can be administered with a needleless hypodermic injection device, such as the devices disclosed in US 5,399,163; US 5,383,851; US 5,312,335; US 5,064,413; US 4,941,880; US 4,790,824; or US 4,596,556. Examples of well-known implants and modules useful in the present invention include those described in: US 4,487,603, which discloses an implantable micro-infusion pump for dispensing medication at a controlled rate; US 4,486,194, which discloses a therapeutic device for administering medicaments through the skin; US 4,447,233, which discloses a medication infusion pump for delivering medication at a precise infusion rate; US 4,447,224, which discloses a variable flow implantable infusion apparatus for continuous drug delivery; US 4,439,196, which discloses an osmotic drug delivery system having multi-chamber compartments; and US 4,475,916, which discloses an osmotic drug delivery system.

Many other such implants, delivery systems, and modules are known to those skilled in the art. In certain embodiments, the compounds according to the present invention can be formulated to ensure proper distribution *in vivo.* For example, the blood-brain barrier (BBB) excludes many highly hydrophilic compounds. To ensure that the compounds according to the present invention cross the BBB (if desired), they can be formulated, for example, in liposomes. For methods of manufacturing liposomes, see, e.g., US 4,522,811; US 5,374,548; and US 5,399,331. The liposomes may comprise one or more moieties which are selectively transported into specific cells or organs, and thus enhance targeted drug delivery (see, e.g., V.V. Ranade (1989) J. Clin. Pharmacol. 29: 685). Exemplary targeting moieties include folate or biotin (see, e.g., US 5,416,016 to Low et al.); mannosides (Umezawa et al., (1988) Biochem. Biophys. Res. Commun. 153: 1038); antibodies (P.G. Bloeman et al. (1995) FEBS Lett. 357: 140; M. Owais et al. (1995) Antimicrob. Agents Chemother. 39: 180); and surfactant protein A receptor (Briscoe et al. (1995) Am. J. Physiol. 1233: 134).

In one embodiment, the compounds according to the present invention (or the compounds used in the present invention) are formulated in liposomes. In a more preferred embodiment, the liposomes include a targeting moiety. In a most preferred embodiment, the compounds in the liposomes are delivered by bolus injection to a site proximal to the desired area. Such liposome-based composition should be fluid to the extent that easy syringability exists, should be stable under the conditions of manufacture and storage and should be preserved against the contaminating action of microorganisms such as bacteria and fungi.

A "therapeutically effective dosage" for therapy/treatment can be measured by objective responses which can either be complete or partial. A complete response (CR) is defined as no clinical, radiological or other evidence of a condition, disorder or disease. A partial response (PR) results from a reduction in disease of greater than 50%. Median time to progression is a measure that characterizes the durability of the objective tumor response.

A "therapeutically effective dosage" for therapy/treatment can also be measured by its ability to stabilize the progression of a condition, disorder or disease. The ability of a compound to inhibit one or more protein kinases or to reduce the viability of cells associated with a proliferative disorder, such as cancer cells can be evaluated by using appropriate *in-vitro* assays known to the skilled practitioner, such as those described herein (in particular in the Examples below). Alternatively, the properties of a compound described in the present invention can be evaluated by examining the ability of the compound in appropriate animal model systems known to the skilled practitioner such as those described herein (in particular in the Examples below). A therapeutically effective amount of a compound according to the present invention can cure, heal, alleviate, relieve, alter, remedy, ameliorate, improve or affect the condition, disorder or disease or the symptoms of the condition, disorder or disease or the predisposition toward the condition, disorder or disease in an individual. One of ordinary skill in the art would be able to determine such amounts based on such factors as the individual's size, the severity of the individual's symptoms, and the particular composition or route of administration selected.

The pharmaceutical composition according to the invention can also, if desired, be presented in a pack, or dispenser device which can contain one or more (e.g., unit) dosage forms containing the active compound. The pack can for example comprise metal or plastic foil, such as blister pack. The pack or dispenser device can be accompanied with a leaflet or other information; in particular, that describing (either to the patient and/or the administering physician) salient information or details on the pharmaceutical composition contained in the package, such as how to administer, recommended dosages, safety and/or side-effect information.

In a particular embodiment, a pharmaceutical composition of the invention is formulated for oral administration, and in an alternative particular embodiment, a pharmaceutical composition of the invention is formulated for intravenous administration.

In one embodiment, a pharmaceutical composition of the invention is in unit dose form, and in particular may be in a unit dose form that is formulated for oral administration.

Each of such a unit dose form may comprise (e.g., it may contain) between 1 and 950mg of the compound, such as the SIK3 kinase inhibitor of the first aspect or a compound used in the fifth aspect, complex, polymorph, crystalline form, racemic mixture, diastereomer, enantiomer, tautomer, conformer, isotopically labeled form, prodrug, or combination thereof. In particular, (e.g., each of) such a unit dose form may comprise (e.g., it may contain) between 2 and 150mg of such compound; and suitably, between 10 and 150mg of such compound.

In particular of such embodiments, a pharmaceutical composition of the invention that is in unit dose form (and in particular one be in a unit dose form that is formulated for oral administration) may comprise (e.g., it may contain) - for each unit dose form - about an amount of such compound selected from the list of amounts consisting of: 2mg, 5mg, 15mg, 20mg, 50mg, 70mg, 80mg, 100mg and 140mg; in particular, comprising (e.g., containing) an amount of about 20mg, 50mg, 70mg or 100mg of a compound of the invention (or of a compound used in the invention).

In one particular embodiment, the pharmaceutical composition of the invention is (e.g., is formed as) a tablet, caplet or capsule; suitably the pharmaceutical composition of the invention (e.g., a unit dose form thereof) is a caplet. Methods to form (e.g., manufacture) tablets and caplets are, for example, described elsewhere herein.

Suitable excipients for the pharmaceutical compositions of the invention, in particular when formed as a tablet or caplet, include, and particular embodiments of such a pharmaceutical composition of the invention include those that further comprise one or more (e.g., all of) the excipients selected from the list consisting of: lactose (e.g., lactose monohydrate), microcrystalline cellulose, croscarmellose sodium, hydroxypropylcellulose and magnesium stearate.

### Therapeutic and other applications

The present application provides **a compound or composition** as specified above under the heading SIk3 inhibitors and/or PD-1 inhibitors, **or a pharmaceutical composition** as specified above under the heading "Pharmaceutical compositions" **for use as a medicament,** for example for use in therapy, in a coadministration, or combination therapy in a subject suffering from a PD-1 therapy resistant tumour.

Treatment including or utilising such compounds may be provided at home, the doctor's office, a clinic, a hospital's outpatient department, or a hospital. Treatment generally begins under medical supervision so that medical personnel can observe the treatment's effects closely and make any adjustments that are needed. The duration of the treatment depends on the age and condition of the patient, as well as how the patient responds to the treatment.

A person having a greater risk of developing a condition, disorder or disease may receive prophylactic treatment to inhibit or delay symptoms of the condition, disorder or disease.

The term "treatment" is known to the person of ordinary skill, and includes the application or administration of a therapeutic agent (e.g., a pharmaceutical composition containing said agent) or procedure to a patient or application or administration of a therapeutic agent (e.g., a pharmaceutical composition containing said agent) or procedure to a cell, cell culture, cell line, sample, tissue or organ isolated from a patient, who has a condition, disorder or disease, a symptom of the condition, disorder or disease or a predisposition toward a condition, disorder or disease, with the purpose to cure, heal, alleviate, relieve, alter, remedy, ameliorate, improve, affect or prevent the condition, disorder or disease, the symptoms of the condition, disorder or disease or the predisposition toward the condition, disorder or disease. Hence, the term "treatment" can include prophylactic treatment of a condition, disorder or disease, or the symptom of a condition, disorder or disease. A therapeutic agent, when used in treatment, includes the kinase inhibitors of the invention (or the compounds used in the fifth aspect of the present invention) and includes, but is not limited to, additional therapeutic agents that may be small molecules, peptides, peptidomimetics, polypeptides/proteins, antibodies, nucleotides such as DNA or RNA, cells, viruses, ribozymes, siRNA, and antisense oligonucleotides.

The disease, disorder or a condition, in the context of the herein described invention, is, in certain embodiments, a proliferative disorder (including a condition or symptom associated with such disorder).

A "proliferative disorder" refers to a disorder characterised by abnormal proliferation of cells. A proliferative disorder does not imply any limitation with respect to the rate of cell growth, but merely indicates loss of normal controls that affect growth and cell division. Thus, in some embodiments, cells of a proliferative disorder can have the same cell division rates as normal cells but do not respond to signals that limit such growth. Within the ambit of "proliferative disorder" is neoplasm or tumour, which is an abnormal growth of tissue or cells. Cancer is art understood, and includes any of various malignant neoplasms characterised by the proliferation of cells that have the capability to invade surrounding tissue and/or metastasise to new colonisation sites. Proliferative disorders include cancer, atherosclerosis, rheumatoid arthritis, idiopathic pulmonary fibrosis and cirrhosis of the liver. Non-cancerous proliferative disorders also include hyperproliferation of cells in the skin such as psoriasis and its varied clinical forms, Reiter's syndrome, pityriasis rubra pilaris, and hyperproliferative variants of disorders of keratinisation (e.g., actinic keratosis, senile keratosis), scleroderma, and the like.

A preferred tumour is a solid tumour. In another particular embodiment the proliferative disorder is (eg, the subject suffers from, or is suspected of suffering from) a solid tumour being one of those described elsewhere herein, such as pancreatic cancer, breast cancer, lung, prostate, melanoma, ovarian cancer, oesophageal cancer, sarcoma and colorectal cancer. In a certain of such embodiments, the proliferative disorder is (eg, the subject suffers from. or is suspected of suffering from) pancreatic cancer; in another of such embodiments, the proliferative disorder is (eg, the subject suffers from, or is suspected of suffering from) prostate cancer; and in yet another of such embodiments, the proliferative disorder is (eg, the subject suffers from, or is suspected of suffering from) lung cancer (eg, non-small cell lung cancer).

In accordance with the present invention, a proliferative disorder, or tumour, treatable with the compounds/compositions of the invention is preferably a tumour by repolarization of the tumour microenvironment, such as by reducing regulatory T cells (Treg) and immunosuppressive myeloid cells and activating the cytotoxic T lymphocyte (CTL) activity together with promotion of the CTL to Treg ratio in the tumour.

In more particular embodiments, the proliferative disorder is a cancer or tumour, in particular a solid tumour (including a condition or symptom associated with such cancer or tumour). Preferred tumours treatable according to the invention are tumours resistant to PD-1 inhibitor therapy, such as a tumour in a patient that received previously a PD-1 inhibitor therapy and which tumour is recurrent and may be in need of additional therapy. The invention provides overcoming PD-1 inhbitor therapy by providing a combination of SIK3 inhbitor therapy with PD-1 inhibitor therapy in accordance with the invention.

According to a further aspect and advantageously in relation to the treatment of a cancer, the invention concerns a composition comprising an SIK3 inhibitor for use in increasing the sensitivity of a subject to a PD-1 inhibitor therapy. As used therein, the expression "resistant to PD-1 inhibitors" can refer to the fact that: the majority of the patients having a given disease do not respond to these treatments (PD-1 inhibitors) and/or have a poor prognostic. In that case, the disease is considered to be globally resistant to anti-PDI treatments, whereas other diseases are sensitive to said treatments. As an example, certain types of cancer are known to be more resistant than others to PD-1 inhibitors; and/or even if a disease is globally known to be sensitive to anti-PDI treatments, a given patient having said disease can be resistant to anti-PD-1 therapies.

According to another embodiment, a patient being resistant to PD-1 inhbitor therapy can be a patient with hyperprogressive disease (HPD) following anti PD-1 treatment, i.e. with accelerated disease upon treatment with PD-1 inhibitors. A further aspect of the invention concerns a method for enhancing the potency of a PD-1 inhibitor administered to a patient as part of a treatment regimen, the method comprising administering to the subject a pharmaceutically effective amount of an SIK3 inhibitor in combination with the PD-1 inhibitor. In other words, the invention concerns a composition comprising an SIK3 inhibitor for use in increasing the efficacy of a treatment with a PD-1 inhibitor in a subject. It further concerns a method of treating a patient in need thereof comprising administering to the patient a therapeutically effective combination of a PD-1 inhibitor with an SIK3 inhibitor, wherein administration of the combination results in enhanced therapeutic efficacy relative to the administration of the PD-1 inhibitor alone.

PD-1- resistant cancers are well known in art. However preferred is a PD-1 resistant non-small cell lung cancer of the squamous cell type.

In certain aspects the invention in view of the forgoing, and the examples below, the invention further pertains to the following preferred itemized aspects and embodiments:
Item 1: **A compound or composition for use in the treatment of a tumour** in a subject, wherein the subject or tumour is resistant to a treatment with an inhibitor of PD-1 or of a ligand thereof; or wherein the subject was treated previously with an inhibitor of PD-1 or of a ligand thereof, and wherein the tumour is recurrent;
   wherein the compound or composition is any one of (i) to (iii):
   (i) a composition comprising the inhibitor of PD-1 or of a ligand thereof (PD-1 inhibitor) and the inhibitor of Salt Inducible Kinase 3 (SIK3 inhibitor); or
   (ii) a SIK3 inhibitor, and wherein the treatment comprises a concomitant or sequential administration of an inhibitor of PD-1 or of a ligand thereof; or
   (iii) an inhibitor of PD-1 or of a ligand thereof, and wherein the treatment comprises a c simultaneous, separate or sequential administration of a SIK3 inhibitor.
Item 2: The compound or composition for use of item 1, wherein the tumour disease is a solid tumour or liquid tumor.
Item 3: The compound or composition for use of item 1 or 2, wherein the is tumour is a recurrent and/or metastatic tumour.
Item 4: The compound or composition for use of any one of items 1 to 3 wherein the treatment is for increasing the sensitivity of the subject to the inhibitor of PD-1 or of a ligand thereof.
Item 5: The compound or composition for use of any one of items 1 to 5, wherein the tumour is selected from (i) a lung cancer of the squamous cell type, preferentially wherein the tumour is a non-small cell lung cancer of the squamous type, or (ii) lymphoma, such as Mediastinal Large B-Cell Lymphoma (PMBCL).
Item 6: The compound or composition for use of items 1 to 5, wherein the inhibitor of PD-1 or of a ligand thereof is an inhibitor of PD-1 or of PD-L1or of PDL2, preferentially an anti-PD-1 antibody or an anti-PD-Ll antibody.
Item 7: The compound or composition for use of item 6, wherein the inhibitor of PD-1 or of a ligand thereof is selected from the group consisting of Pembrolizumab, Nivolumab, BMS-936559, Cemiplimab, Avelumab, Durvalumab, Atezolizumab, Dostarlimab, Spartalizumab, or a combination thereof.
Item 8: The compound or composition for use of any one of items 1 to 7, wherein the SIK3 inhibitor is a compound selected from a compound selected from the group consisting of a kinase inhibitor of the formula: 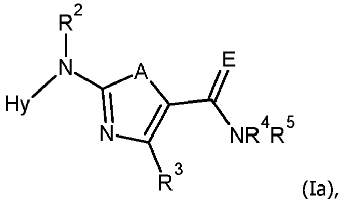 and solvates, salts, N-oxides, complexes, polymorphs, crystalline forms, racemic mixtures, diastereomers, enantiomers, tautomers, conformers, isotopically labeled forms, prodrugs, and combinations thereof; wherein Hy, R2, R3, R4, R5, A, and E are as in WO 2021/214117.
Item 9: The compound or composition for use of item 8, wherein the SIK3 inhibitor is a compound having the formula: 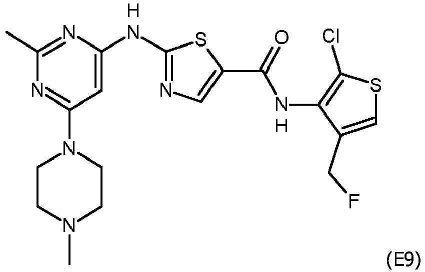 (*N*-(2-chloro-4-(fluoromethyl)thiophen-3-yl)-2-((2-methyl-6-(4-methylpiperazin-1-yl)pyrimidin-4-yl)amino)thiazole-5-carboxamide),
   and solvates, salts, N-oxides, complexes, polymorphs, crystalline forms, racemic mixtures, diastereomers, enantiomers, tautomers, conformers, isotopically labeled forms, prodrugs, and combinations thereof.
Item 10:The compound or composition for use of any one of the preceding items, which is in the form of a preparation for simultaneous, separate or sequential use in therapy in the subject.
Item 11:The compound or composition for use of item 10, wherein the preparation comprises the inhibitor of PD-1 or of a ligand thereof and the SIK3 inhibitor.
Item **12:A pharmaceutical composition** comprising a inhibitor of PD-1 or of a ligand thereof (PD-1 inhibitor) and an inhibitor of Salt Inducible Kinase 3 (SIK3 inhibitor), together with a pharmaceutical acceptable carrier and/or excipient.
Item **13:A pharmaceutical kit,** comprising an inhibitor of PD-1 or of a ligand thereof and a SIK3 inhibitor, wherein the kit is prepared to allow for a simultaneous, separate, or sequential administration of the PD-1 inhibitor and the SIK3 inhibitor in therapy to a subject.
Item 14:The pharmaceutical composition of item 12 or 14, or the pharmaceutical kit of item 13 or 14, wherein the PD-1 inhibitor and/or the SIK3 inhibitor is as recited in any one of items 1 to 11.
Item 15:The pharmaceutical composition of item 12, 14 or 15, or the pharmaceutical kit of item 13, 14 or 15, for use in a treatment as recited in any one of items 1 to 11.
Item 16:**A method for treating a tumour disease** in a subject, wherein the subject or tumour is resistant to a treatment with an inhibitor of PD-1 or of a ligand thereof; or wherein the subject was treated previously with an inhibitor of PD-1 or of a ligand thereof, and wherein the tumour is recurrent;
   wherein the method comprises a step (i) or (ii):
   (i) administration of a therapeutically effective amount of a composition which comprises the inhibitor of PD-1 or of a ligand thereof (PD-1 inhibitor) and the inhibitor of Salt Inducible Kinase 3 (SIK3 inhibitor) to the subject; or
   (ii) administration of a therapeutically effective amount of a SIK3 inhibitor to the subject, and wherein the treatment further comprises a simultaneous, separate or sequential administration of an inhibitor of PD-1 or of a ligand thereof.
Item 17:The method of item 16, wherein the tumour disease is a solid tumour.
Item 18:The method of item 16 or 17, wherein the is tumour is a recurrent and/or metastatic tumour.
Item 19:The method of any one of items 16 to 18, wherein the treatment is for increasing the sensitivity of the subject to a therapy with the inhibitor of PD-1 or of a ligand thereof.
Item 20:The method of any one of items 16 to 19, wherein the tumour is selected from (i) a lung cancer of the squamous cell type, preferentially wherein the tumour is a non-small cell lung cancer of the squamous type, or (ii) lymphoma, such as Mediastinal Large B-Cell Lymphoma (PMBCL).
Item 21:The method of any one of items 16 to 20, wherein the inhibitor of PD-1 or of a ligand thereof is an inhibitor of PD-1 or of PD-L1 or of PDL2, preferentially an anti-PD-1 antibody or an anti-PD-Ll antibody.
Item 22:The method of any one of items 16 to 21, wherein the inhibitor of PD-1 or of a ligand thereof is selected from the group consisting of Pembrolizumab, Nivolumab, BMS-936559, Cemiplimab, Avelumab, Durvalumab, Atezolizumab, Spartalizumab, Dostarlimab or a combination thereof.
Item 23:The method of any one of items 16 to 22, wherein the SIK3 inhibitor is a compound selected from a compound selected from the group consisting of a kinase inhibitor of the formula: 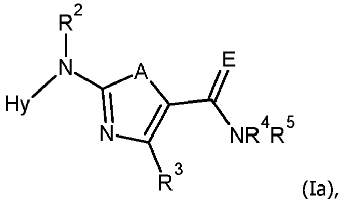 and solvates, salts, N-oxides, complexes, polymorphs, crystalline forms, racemic mixtures, diastereomers, enantiomers, tautomers, conformers, isotopically labeled forms, prodrugs, and combinations thereof; wherein Hy, R2, R3, R4, R5, A, and E are defined as for the compound of formula (Ia) in WO 2021/214117.
Item 24:The method of item 23, wherein the SIK3 inhibitor is a compound having the formula: 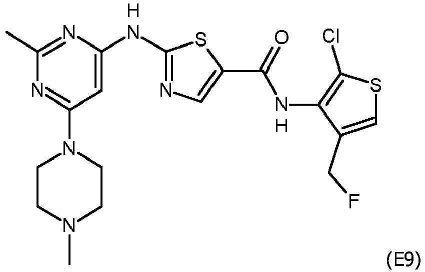 (*N*-(2-chloro-4-(fluoromethyl)thiophen-3-yl)-2-((2-methyl-6-(4-methylpiperazin-1-yl)pyrimidin-4-yl)amino)thiazole-5-carboxamide),
   and solvates, salts, N-oxides, complexes, polymorphs, crystalline forms, racemic mixtures, diastereomers, enantiomers, tautomers, conformers, isotopically labeled forms, prodrugs, and combinations thereof.

In view of the above, it will be appreciated that the present invention also relates to the following itemised embodiments:

### EXAMPLES

The examples show:

### Example 1: SIK3 inhibitor E9 repolarizes the tumor microenvironment and shows strong anti-tumor efficacy in monotherapy and in combination with PD-1 blockade

MC38 tumor cells were subcutaneously implanted in C57BI6/N mice, randomized at an average tumor volume of 100 mm³ and tumor bearing mice were treated twice daily with 25 mg/kg compound E9 by oral gavage and twice weekly, intraperitoneal with 10 mg/kg anti-murine-PD-1 mAb (clone: RMP1-14) or corresponding controls. Results are shown in Figure 3A and 3B.

Figure 3 shows that the SIK3 inhibitor E9 demonstrated highly significant anti-tumor efficacy and prolonged survival in monotherapy in the MC38 tumor model. Further, therapy with E9 repolarized the tumour microenvironment by reducing regulatory T cells (Treg) and immunosuppressive myeloid cells and activating the cytotoxic T lymphocyte (CTL) activity together with promotion of the CTL to Treg ratio. The SIK3 inhibitor E9 in combination with anti-PD-1 exploited the full potential of the anti-tumor immune environment

### Example 2: SIK3 inhibitor E9 acts synergistically with PD-1 blockade in immune checkpoint resistant breast and lung cancer models

EMT6 tumor cells were implanted into mammary fat pad in BALB/c mice and randomized at an average tumor volume of 65mm³. KLN205 tumor cells were subcutaneously implanted in DBA/2 mice and randomized at an average tumor volume of 60 mm³. Both animal models were treated twice daily by oral gavage with the indicated doses of compound E9 and twice weekly, intraperitoneal with 10 mg/kg anti-murine-PD-1 mAb (clone: RMP1-14) or corresponding controls. Results are shown in Figures 4A and 4B.

Figure 4A shows that a combination therapy of SIK3 inhibitor (E9) and PD-1 blockade acted synergistically in the immune excluded breast cancer model EMT6 and the immune checkpoint resistant lung squamous cell carcinoma model KLN205. Surprisingly a complete tumor regression and extension of overall survival were achieved in EMT6 tumors and significantly delayed tumor outgrowth in KLN205 lung cancer (Figure 4B). In particular in the murine lung squamous cell carcinoma cell line KLN205 a surprising synergy is observed when combining compound E9 with anti PD-1 antibodies. Growth inhibition for single treatments increased from -6%TGI for PD-1 alone (compared to control) and 22% for E9 (compared to control) to 44% increase of TGI for the combination.

## Claims

1. **A compound or composition for use in the treatment of a tumour** in a subject, wherein the subject or tumour is resistant to a treatment with an inhibitor of PD-1 or of a ligand thereof; or wherein the subject was treated previously with an inhibitor of PD-1 or of a ligand thereof, and wherein the tumour is recurrent; wherein the compound or composition is any one of (i) to (iii):
(i) a composition comprising the inhibitor of PD-1 or of a ligand thereof (PD-1 inhibitor) and the inhibitor of Salt Inducible Kinase 3 (SIK3 inhibitor); or
(ii) a SIK3 inhibitor, and wherein the treatment comprises a concomitant or sequential administration of an inhibitor of PD-1 or of a ligand thereof; or
(iii) an inhibitor of PD-1 or of a ligand thereof, and wherein the treatment comprises a c simultaneous, separate or sequential administration of a SIK3 inhibitor.

2. The compound or composition for use of claim 1, wherein the tumour disease is a solid tumour or liquid tumor.

3. The compound or composition for use of claim 1 or 2, wherein the is tumour is a recurrent and/or metastatic tumour.

4. The compound or composition for use of any one of claims 1 to 3 wherein the treatment is for increasing the sensitivity of the subject to the inhibitor of PD-1 or of a ligand thereof.

5. The compound or composition for use of any one of claims 1 to 5, wherein the tumour is selected from (i) a lung cancer of the squamous cell type, preferentially wherein the tumour is a non-small cell lung cancer of the squamous type, or (ii) lymphoma, such as Mediastinal Large B-Cell Lymphoma (PMBCL).

6. The compound or composition for use of claims 1 to 5, wherein the inhibitor of PD-1 or of a ligand thereof is an inhibitor of PD-1 or of PD-L1 or of PDL2, preferentially an anti-PD-1 antibody or an anti-PD-Ll antibody.

7. The compound or composition for use of claim 6, wherein the inhibitor of PD-1 or of a ligand thereof is selected from the group consisting of Pembrolizumab, Nivolumab, BMS-936559, Cemiplimab, Avelumab, Durvalumab, Atezolizumab, Dostarlimab, Spartalizumab, or a combination thereof.

8. The compound or composition for use of any one of claims 1 to 7, wherein the SIK3 inhibitor is a compound selected from a compound selected from the group consisting of a kinase inhibitor of the formula: 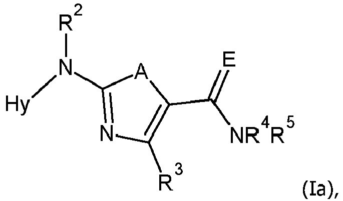 and solvates, salts, N-oxides, complexes, polymorphs, crystalline forms, racemic mixtures, diastereomers, enantiomers, tautomers, conformers, isotopically labeled forms, prodrugs, and combinations thereof; wherein Hy, R2, R3, R4, R5, A, and E are as in WO 2021/214117.

9. The compound or composition for use of claim 8, wherein the SIK3 inhibitor is a compound having the formula: 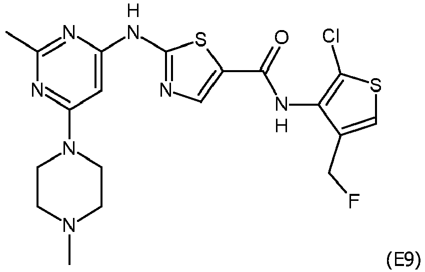 (*N*-(2-chloro-4-(fluoromethyl)thiophen-3-yl)-2-((2-methyl-6-(4-methylpiperazin-1-yl)pyrimidin-4-yl)amino)thiazole-5-carboxamide),
and solvates, salts, N-oxides, complexes, polymorphs, crystalline forms, racemic mixtures, diastereomers, enantiomers, tautomers, conformers, isotopically labeled forms, prodrugs, and combinations thereof.

10. The compound or composition for use of any one of the preceding claims, which is in the form of a preparation for simultaneous, separate or sequential use in therapy in the subject.

11. The compound or composition for use of claim 10, wherein the preparation comprises the inhibitor of PD-1 or of a ligand thereof and the SIK3 inhibitor.

12. **A pharmaceutical composition** comprising a inhibitor of PD-1 or of a ligand thereof (PD-1 inhibitor) and an inhibitor of Salt Inducible Kinase 3 (SIK3 inhibitor), together with a pharmaceutical acceptable carrier and/or excipient.

13. **A pharmaceutical kit,** comprising an inhibitor of PD-1 or of a ligand thereof and a SIK3 inhibitor, wherein the kit is prepared to allow for a simultaneous, separate, or sequential administration of the PD-1 inhibitor and the SIK3 inhibitor in therapy to a subject.

14. The pharmaceutical composition of claim 12 or 14, or the pharmaceutical kit of claim 13 or 14, wherein the PD-1 inhibitor and/or the SIK3 inhibitor is as recited in any one of claims 1 to 11.

15. The pharmaceutical composition of claim 12, 14 or 15, or the pharmaceutical kit of claim 13, 14 or 15, for use in a treatment as recited in any one of claims 1 to 11.
